# FASCICULE DE BREVET EUROPEEN

(11) **EP 0 833 917 B1**
(45) Date de publication et mention de la délivrance du brevet: **17.08.2005**
(21) Numéro de dépôt: 96922107.6
(22) Date de dépôt: 12.06.1996
(51) Int. Cl.: C12N 15/30, C07K 14/445, C07K 16/20, A61K 39/015, A61K 39/395, A61K 39/385, G01N 33/68, C12N 15/70, C07K 17/08

(54) **MOLECULES POLYPEPTIDIQUES DE STADE PRE-ERYTHROCYTAIRE DU PALUDISME**
GEGEN MALARIA POLYPEPTIDISCHE MOLEKULEN VON VORERYTHROZYTÄREN STADIUM
MALARIAL PRE-ERYTHROCYTIC STAGE POLYPEPTIDE MOLECULES

(30) Priorité: 13.06.1995 FR 9507007
(43) Date de publication de la demande: 08.04.1998
(73) Titulaire: INSTITUT PASTEUR, 75724 Paris Cédex 15 (FR)
(72) Inventeur: DRUILHE, Pierre, 75016 Paris (FR); DAUBERSIES, Pierre, 75015 Paris (FR)
(74) Mandataire: Becker, Philippe
(86) Numéro de dépôt international: PCT/FR1996/000894
(87) Numéro de publication internationale: WO 1996/041877

(56) Documents cités:
- WO-A-92/13884
- WO-A-94/09140
- FR-A- 2 679 909
- MOLECULAR AND BIOCHEMICAL PARASITOLOGY 64 (2). 1994. 219-232, XP000611837 FIDOCK D A ET AL: "Cloning and characterization of a novel Plasmodium falciparum sporozoite surface antigen, STARP."

## Description

Les parasites responsables du paludisme chez l'homme présentent chez l'hôte humain des morphologies différentes et expriment des antigènes différents en fonction de leur localisation dans l'organisme. Les différences morphologiques et antigéniques de ces parasites au cours de leurs cycles de vie chez l'homme, permettent de définir différents stades de développement dans le foie et dans le sang : le sporozoïte, forme infectieuse injectée par le moustique vecteur, se transforme rapidement en schizonte dans les hépatocytes de l'hôte pour infecter ensuite les erythrocytes. La localisation intra-hépatique dé P.falciparum se traduit par l'expression d'un groupe d'antigènes spécifiques de ce stade de développement et très immunogènes dans les condition naturelles d'exposition à la maladie. Cette phase, cliniquement silencieuse, est actuellement la seule contre laquelle on peut induire expérimentalement chez l'homme une immunité très forte, stérilisante, par injection de sporozoïtes irradiés, capables de pénétrer dans l'hépatocyte et de s'y développer, mais sans pouvoir aboutir au stade sanguin de la maladie. C'est pourquoi les inventeurs ont focalisé l'essentiel de leurs efforts sur ces deux stades pré-érythrocytaires. Mais ces stades sont également les plus délicats à étudier, et donc les moins connus, puisque l'obtention de matériel biologique est difficile, voire impossible, que le seul modèle d'étude in vitro possède un rendement très faible et que le meilleur modèle animal reste le chimpanzé, d'utilisation limitée et onéreuse.

Afin d'accéder aux antigènes des stades pré-érythrocytaires, les inventeurs ont utilisé des sérums d'individus résidant en zone d'endémie depuis 25 ans mais sous prophylaxie permanente à la chlroroquine. Ces individus étaient régulièrement soumis aux piqûres de moustiques infectés mais ne développaient aucune infection sanguine complète. Leur sérum contenait donc des anticorps essentiellement dirigés contre les stades pré-érythrocytaires, ce qui fut vérifié par Immuno-Fluorescence (IF) et Westem Blot sur les 3 stades du parasite.

L'utilisation de ces sérums pour le criblage d'une banque d'ADN génomique du clone parasitaire de P.falciparum, construite en vecteurs d'expression dans un phage lambda gt11 (V. Rosario, Science 212, 1981, p.1037-1038 ; et Thaithong et al, Transactions of Royal Society of Tropical Medicine and Hygien, 1984, 78 : 242-245) a conduit à la mise en évidence de polypeptides du stade pré-erythrocytaire, notamment les polypeptides SALSA (pour Sporozoïte Liver Stage Antigen) décrits dans EP A-0407230 et le LSA 1 (pour Liver Stage Antigen) décrit dans W0 92/13884. La présente invention est relative à de nouvelles molécules polypeptidiques spécifiques du stade pré-érythrocytaire et à leur utilisation à titre de principe actif de vaccin anti-palustre ou dans des méthodes de diagnostics de la maladie.

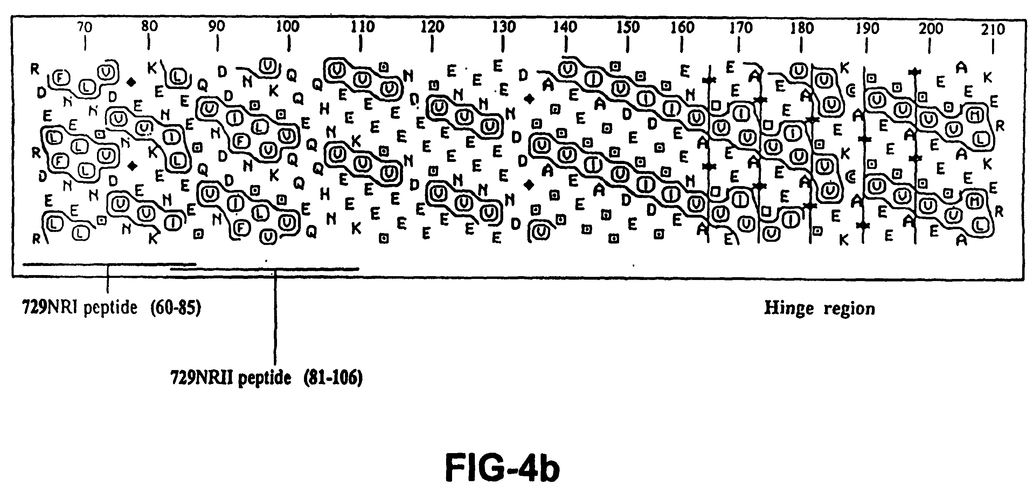

L'invention résulte de la mise en évidence par les inventeurs des propriétés particulières d'un antigène particulier appelé LSA-3 et de ses fragments, qui apparaissent comme des candidats à fort potentiel pour réaliser un vaccin anti-palustre et ceci pour les raisons suivantes :
a) lorsque une fraction de LSA-3 était utilisée en combinaison avec un autre antigène du même stade de développement du parasite, comme le LSA-1, pour immuniser des chimpanzés, l'animal répondant aux deux molécules ou uniquement à LSA-3 présente la caractéristique de ne pas avoir de parasites dans le sang, d'avoir une diminution importante des parasites dans le foie, et de manifester un recrutement de cellules mononucléées important indiquant une réponse en immunité cellulaire ;
b) en zone d'endémie, on observe une très nette corrélation entre la protection des individus contre les infections naturelles par sporozoïtes et leurs réponses en anticorps contre LSA-3 ;
c) chez huit volontaires humains immunisés par injection de sporozoïtes irradiés, des anticorps dirigés contre LSA-3 sont trouvés chez chacun des quatre individus résistant à une infection par sporozoïtes et chez aucun des quatre autres volontaires ayant développé un infection sanguine ;
d) des anticorps obtenus contre le peptide DG729 dans WO 92/13884 déjà décrit donnent une réaction croisée avec les stades sporozoïtes et hépatiques du parasite murin P yoelii ce qui permet une exploitation significative du modèle souris. In vitro les anticorps humains immuno purifiés sur DG729 sont capables, même à très faibles concentrations, de bloquer la pénétration des sporozoïtes de P yoelii dans les hépatocytes murins. In vivo les souris immunisées par DG729 sont totalement ou partiellement protégées contre les infections par les sporozoïtes de P.yoelii;
e) enfin, certains épitopes notamment dans les parties non répétées de la molécule stimulent la sécrétion d'interféron γ par les monocytes, ce médiateur permettant d'inhiber le développement intra-hépatique du parasite (S. Mellouk et al, The Jour. of Imun. 139, 4192-4195, 1987).
f) la séquence de la région de LSA-3 correspondant à un (lipo) peptide NR2 a été analysée dans 27 échantillons : 4 souches de laboratoire (NF54, K1, Palo Alto, T9/96), 3 isolats malgaches, 3 isolats birmans, 5 isolats brésiliens, 7 isolats ivoiriens et 5 isolats thaïs. Aucune mutation n'a été observée sur les 300 paires de bases analysées, soit 100 % de conservation dans cette région immunologiquement importante contenant un ou plusieurs épitopes B, Th et CTL.
g) des informations sur la structure de l'antigène, et notamment d'un peptide RE, et plus particulièrement sur la région répétée centrale à partir de laquelle le peptide RE a été conçu et qui comporte un ou plusieurs épitopes B majeurs, ont été obtenues à partir du Hydrophobic Cluster Plot de la séquence disponible dans le clone T9/96 (630 acides aminés), (Gaboriot et al., (1987) : Hydrophobic cluster analysis : an efficient new way to compare and analyse amino acide sequences, FEBS Letters, 224 : 149-155); cette méthode prédit une très forte propension à l'organisation en hélice-α. La région répétée présente une extraordinaire régularité dans l'espacement des résidus de Valine et d'Isoleucine, alternant avec des résidus acides ou de Proline. La disposition des groupements hydrophobes à la surface de cette hélice évoque une bordure hydrophobe se décalant graduellement d'une face de l'hélice à l'autre, suivant une orientation générale constante le long de la molécule, et probablement en relation avec une structure ou un empaquetage « coiled-coil » comme cela apparaît dans la figure 4b qui représente le HCP (Hydrophobic Cluster Plot) de la séquence peptidique du clone DG729.
h) après avoir démontré qu'il existait une très large gamme de réponses immunitaires à l'antigène LSA-3, nous avons analysé la capacité des cellules répondeuses à se localiser autour des parasites dans le foie. Chez les souris immunisées par les antigènes recombinants, l'injection par voie intraportale de chacun des peptides absorbés sur des billes de polystyrène de 10µm permet de visualiser au bout de 48 heures un afflux de lymphocytes autour de l'antigène (mimant le parasite), puis au 5ème jour, un important recrutement de cellules appartenant à la lignée macrophagique.

Toutes ces propriétés dont certaines seront démontrées en détail dans les expériences décrites plus loin montrent que l'antigène LSA-3 présente à la fois une bonne antigénicité et une bonne immunogénicité.

Les inventeurs ont pu confirmer et préciser la spécificité des stades d'expression de la molécule ; au niveau des sporozoïtes, cette expression a été confirmée par l'immunofluorescence en surface de plusieurs souches et isolats. En analyse par « Western Blot » (ou Immunoempreinte), la molécule LSA-3 apparaît comme une protéine d'un poids moléculaire de 200 000 daltons. Si les ARN messagers de sporozoïtes n'ont pu être obtenus en quantité suffisante pour une analyse en « northern blot », des expériences de PCR inverse ont confirmé l'expression de LSA-3 à ce stade. Au niveau des hépatocytes infectés, LSA-3 est observé dans la vacuole parasitophore du parasite par immunofluorescence à l'aide d'anticorps contre les régions répétées et non répétées de la protéine, ainsi qu'en microscopie électronique.

Un fragment de LSA-3 dénommé 729S ainsi que trois peptides dénommés NRI et NRII inclus dans la partie non répétée, et 729 R inclus dans la partie répétée ont été décrits dans la demande WO 92/13884. Néanmoins ce document n'évoque pas les propriétés particulières mentionnées ci-dessus ni d'autres fragments de LSA-3 qui pourraient être soit plus longs soit plus courts, inclus ou combinés avec ces fragments qui présenteraient des propriétés particulièrement intéressantes pour une utilisation dans des vaccins.

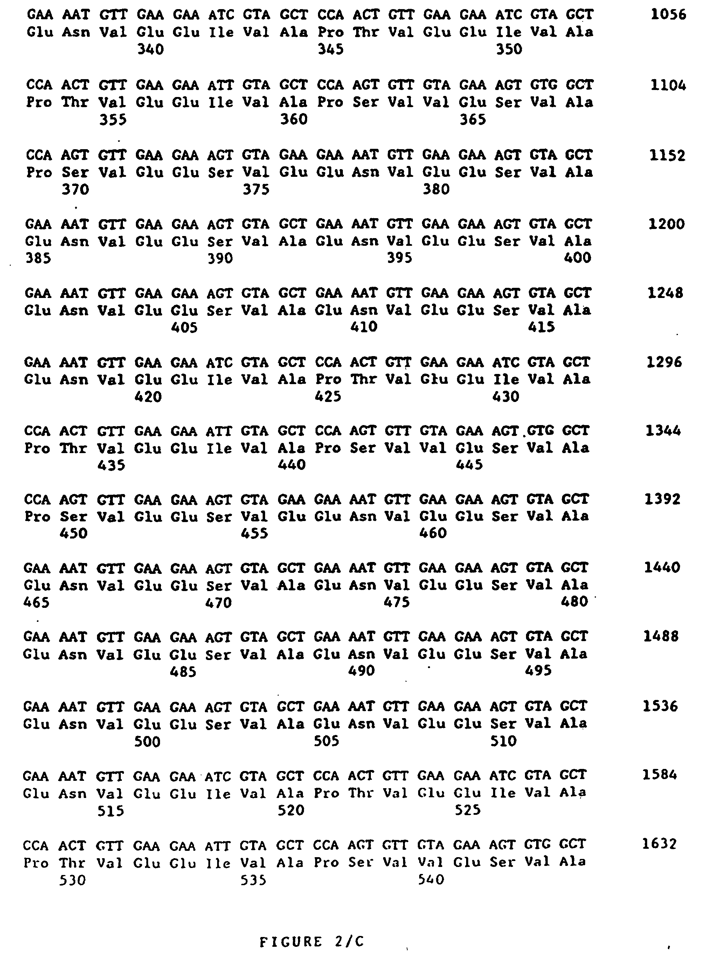

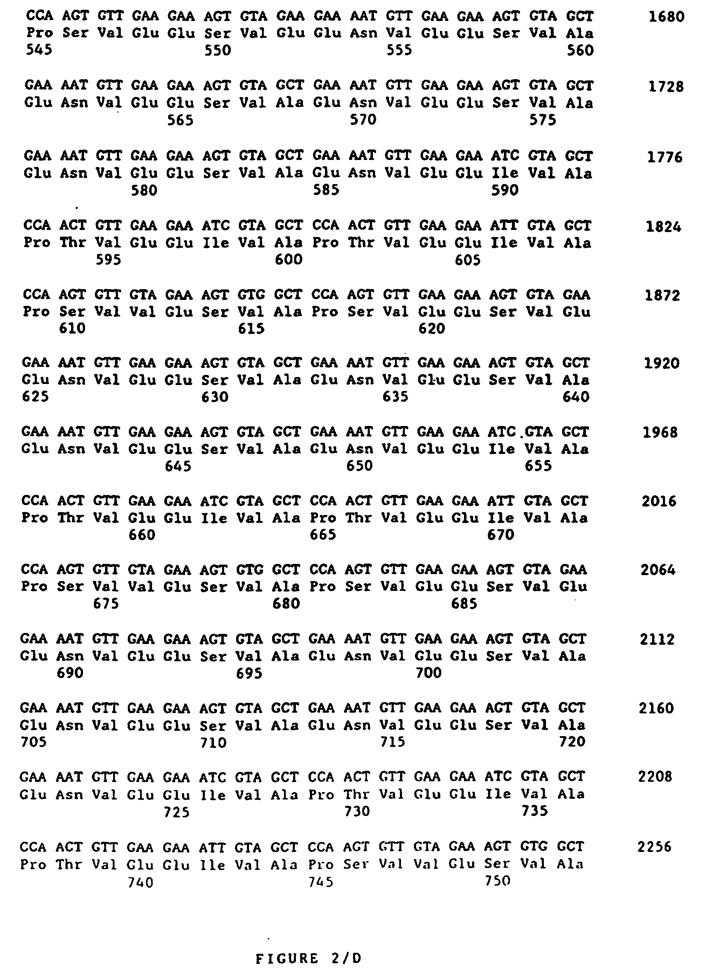

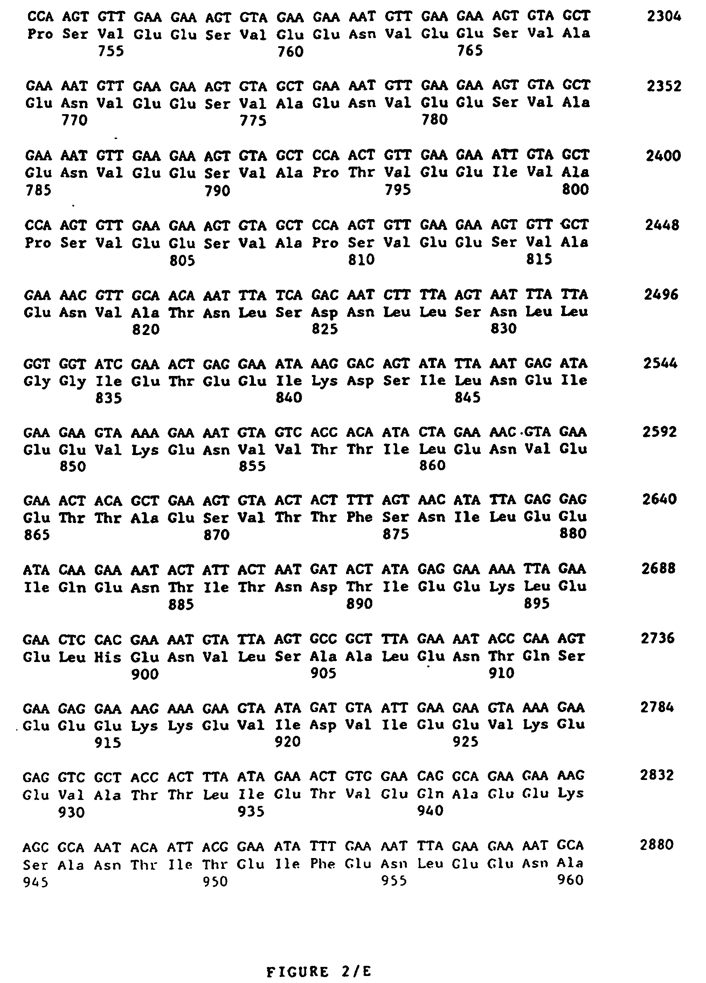

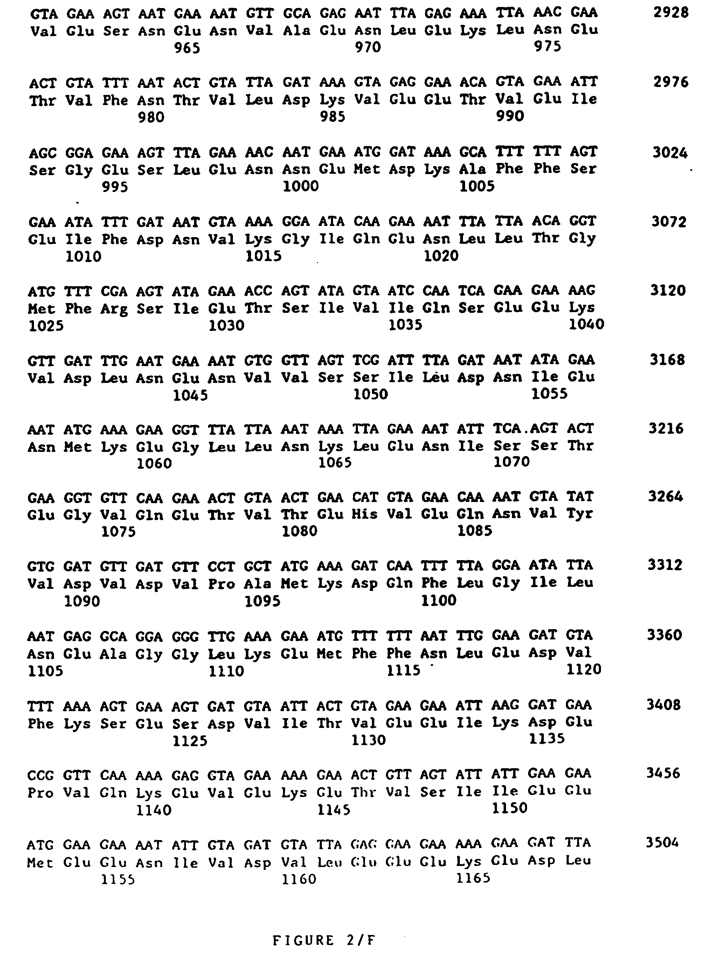

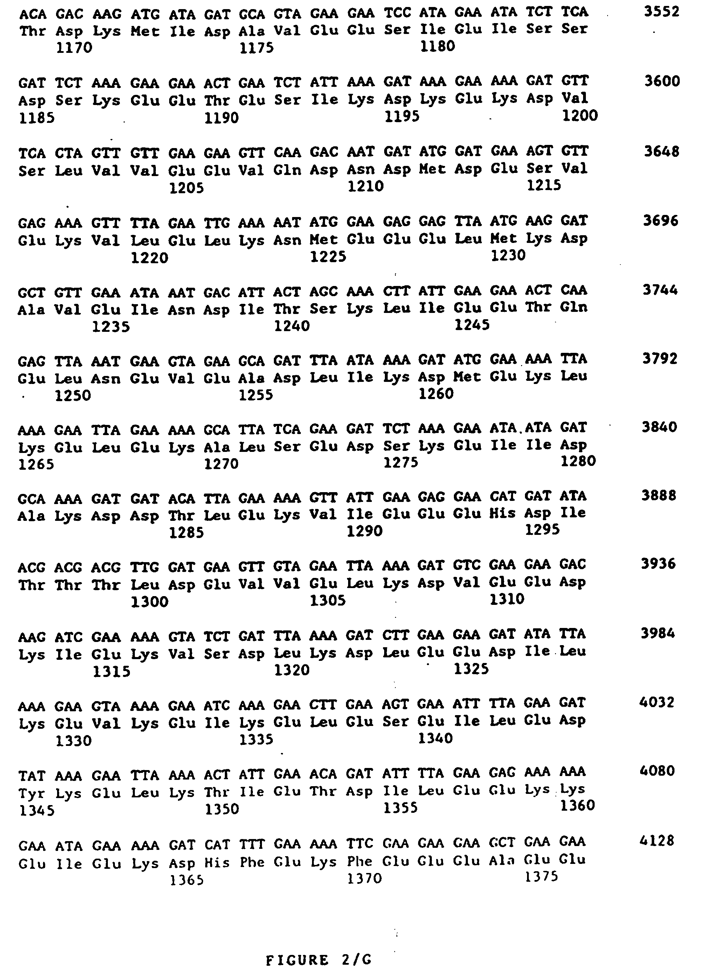

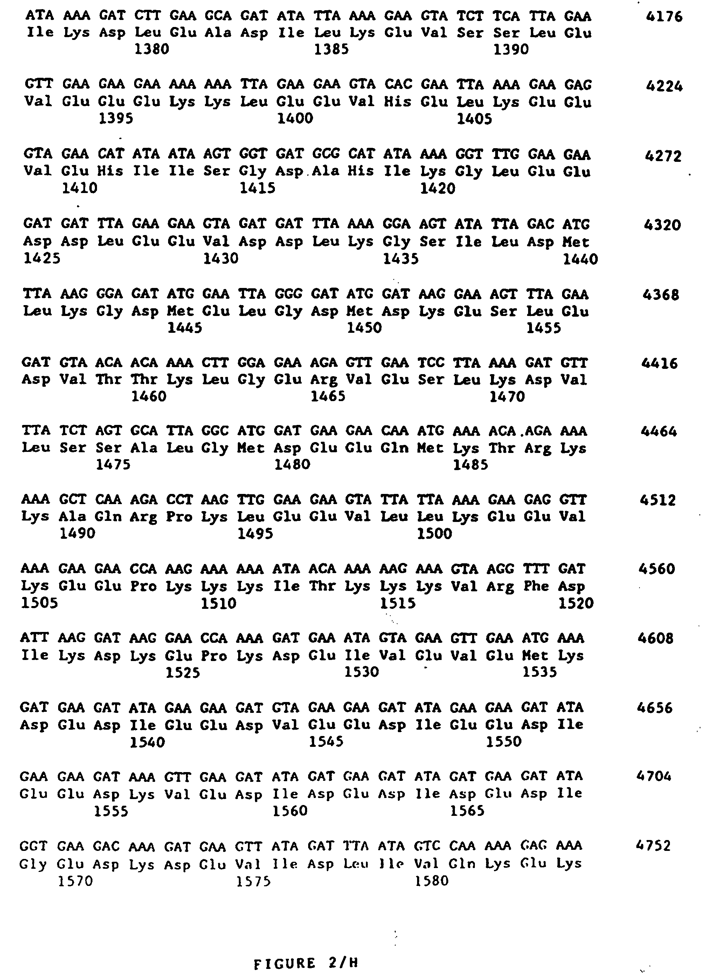

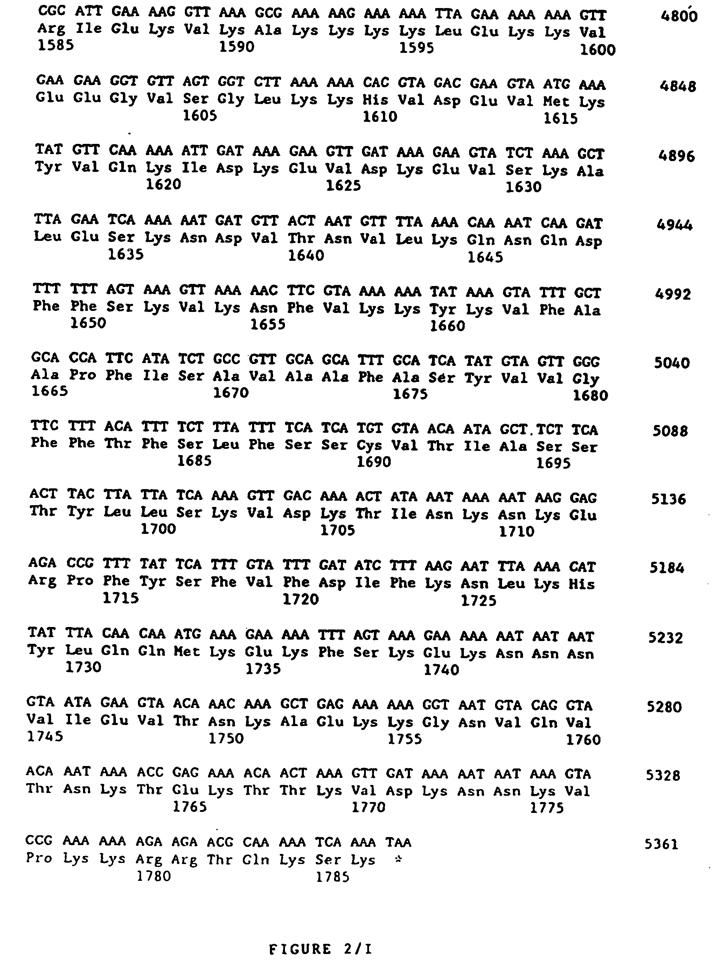

L'invention a pour objet des molécules polypeptidiques contenant au moins dix acides aminés consécutifs de la séquence d'acides aminés montrée dans la figure 2 et dénommée SEQ ID n° 2, et représentant LSA-3, les polypeptides suivants étant exclus :
- RDELFNELLNSVDVNGEVKENILEESQVNDDIFNSLVKSVQQEQQHNVEEK VEESVEENDEESVEENVEENVENNDDGSVASSVEESIASSVDESIDSSIE ENVAPTVEEIVAPTVEEIVAPSWEKCAPSVEESVAPSVEESVAEMLKER (729S)
- RDELFNELLNSVDVNGEVKENILEESQVNDDIFNSLVKSVQQEQQHN
- DELFNELLNSVDVNGEVKENILEESQ, (NR I)
- LEESQVNDDIFSNSLVKSVQQEQQHNV, (NR II)
- VESVAPSVEESVAPSVEESVAENVEESV. (729 RE)

D'autres molécules selon l'invention contiennent au moins 20 acides aminés consécutifs ou au moins 50.

L'ensemble de ces polypeptides et la molécule LSA-3 sont dans tout ce qui suit « polypeptides de l'invention ».

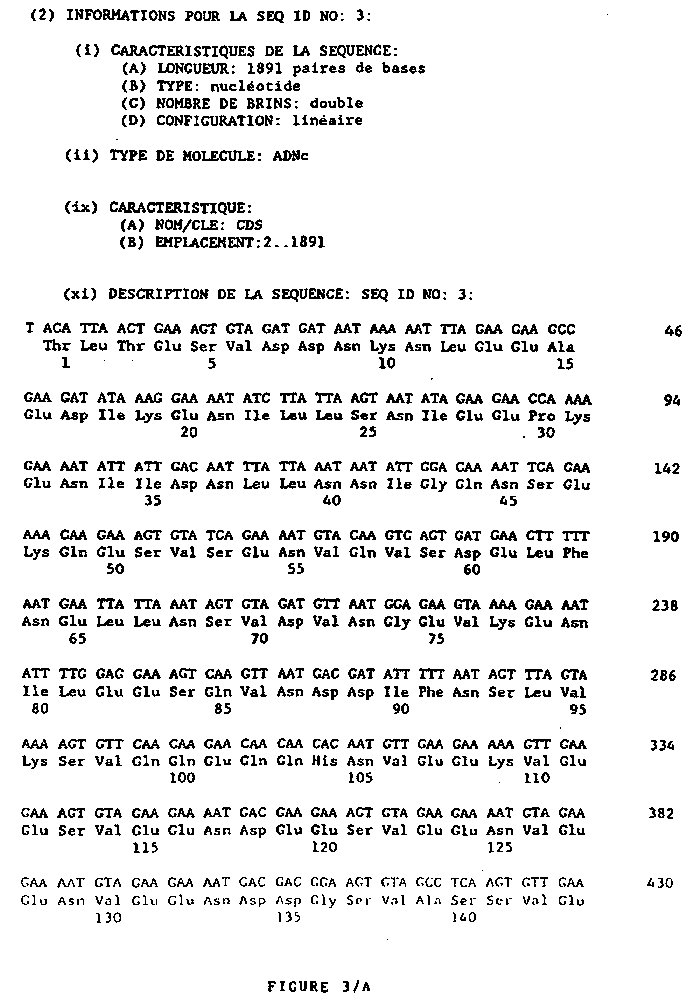

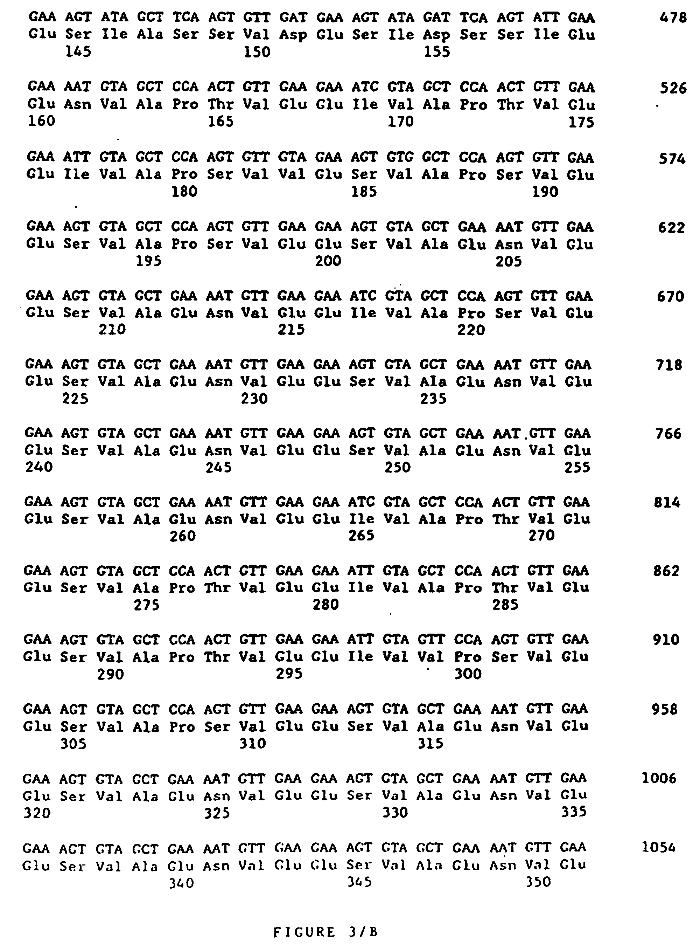

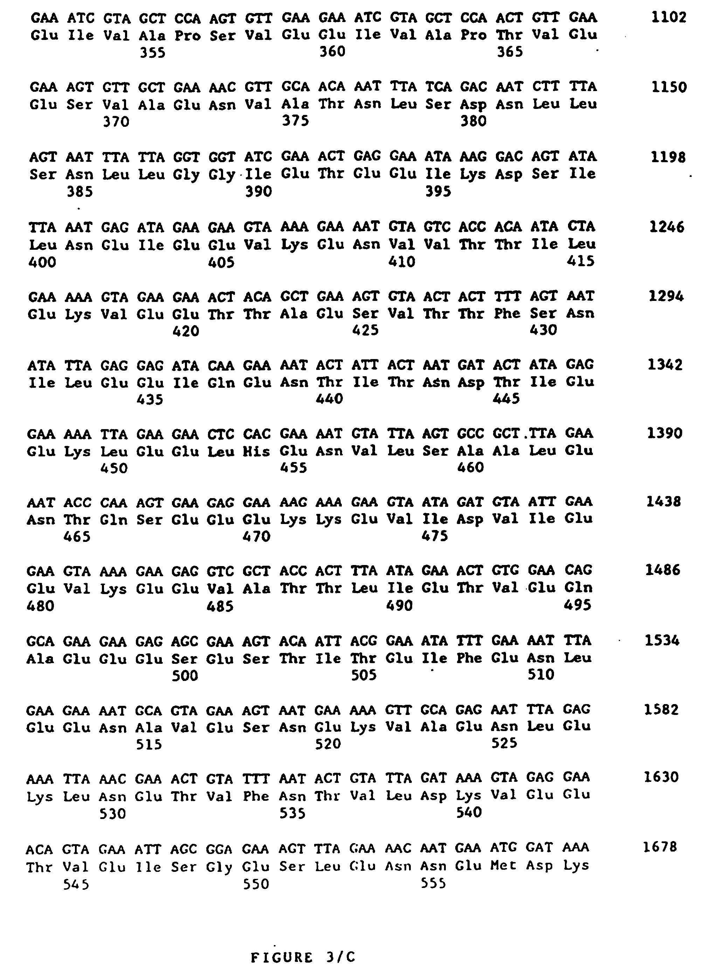

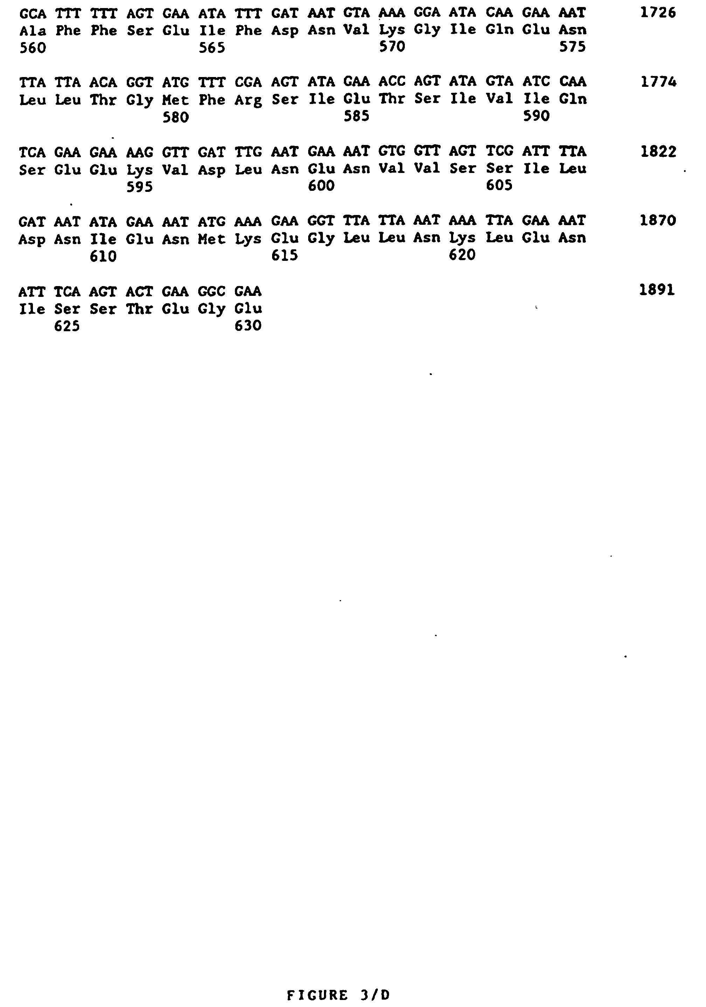

Les résultats expérimentaux et les comparaisons de séquence non répétées entre différents isolats de P.falciparum indiquent l'existence d'une homologie d'au moins 70% entre des antigènes équivalents du stade hépatique du parasite. Aussi toute molécule peptidique présentant au moins 70% d'homologie avec une quelconque des molécules définies ci-dessus fait partie de l'invention, ainsi que celles présentant au 70% d'homologie avec la séquence suivante : comprise entre les acides aminés 140-159 de K1 ou 23-42 du T9/96.
De la même façon font partie de l'invention les molécules polypeptidiques présentant au moins 70% d'homologie avec la séquence représentée dans la figure 3 qui représente une partie de LSA-3 dans T9/96 : l'ADN de cette isolat de P.falciparum a été digéré par des enzymes de restriction puis cloné dans lambda gt11 et a ainsi permis de constituer la génothèque de cette isolat déjà décrite plus haut.

Des conjugués constitués d'un polypeptide issu de LSA-3 lié de façon covalente par un pont lysine à des résidus lipidiques saturés ou insaturés font également partie de l'invention, plus particulièrement lorsque le résidu lipidique est un palmitoyl ou un palmityl ou un oléyl. Des résidus en C16 ou C18 ont ainsi été couplés par un pont lysine aux peptides NRI, NRII, 729 RE et CT1 déjà représentés ci-dessus. La méthode de synthèse utilisée pour ces conjugués est décrite dans Bourgault, Journal of Immunology, 149, 3416 (1992) et Rouaix, Vaccine, 12, 1209 (1994).

L'invention porte également sur des compositions immunogènes contenant au moins une molécule polypeptidique ou un conjugué décrits ci-dessus, ainsi que sur les vaccins contenant ces compositions immunogènes. D'autres épitopes immunogènes notamment LSA-1, SALSA, STARP ont déjà été décrits dans EP A-0407230 et dans WO 92/13884 Les compositions de vaccin selon l'invention peuvent contenir de façon avantageuse un mélange de peptides immunogènes issus de LSA-3 et des peptides ou antigènes issus de LSA-1, SALSA ou STARP ; un mélange plus particulièrement intéressant pourrait être celui constitué d'une part de NRI, NRII ou de LSA-3 entier, couplés ou non à un résidu lipidique, et d'autre part des peptides SALSA-1 ou SALSA2 ou l'antigène SALSA couplé ou non à un résidu lipidique.

Toutes les molécules polypeptidiques répondant à la définition ci-dessus et présentant au moins 70% d'homologie avec les polypeptides LSA-3, CT1, NRI, NRII ou 729RE, peuvent être combinées de façon homologue ou hétérologue avec d'autres séquences peptidiques ou issues d'un autre antigène des différents stades de P.falciparum.

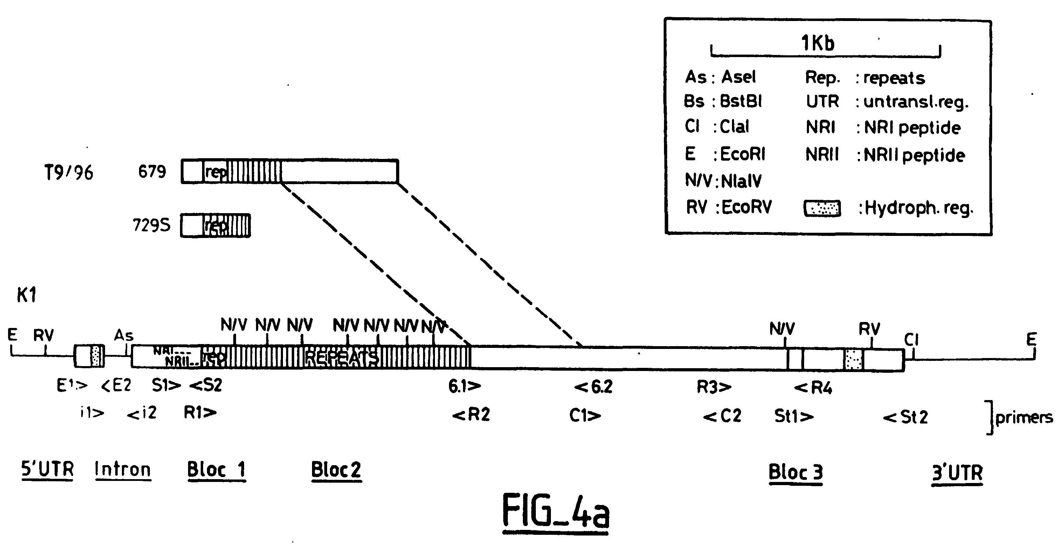

Par 70 % d'homologie des séquences, il est bien entendu qu'il s'agit d'une homologie de séquences par rapport à l'un quelconque des isolats dont la séquence est connue ou susceptible d'être connue, et non pas globalement entre l'ensemble des isolats. En effet, la région répétée centrale de LSA-3 (bloc 2 de la figure 4) présente un nombre variable de séquences répétées responsables d'une variabilité d'un isolat à l'autre comme l'indique d'ailleurs la représentation de la figure 4 dans laquelle la différence de longueur entre les parties répétées du bloc 2 des isolats T9/96 et K1 est flagrante bien que les tétrapeptides qui constituent cette région répétée (VEES, VEEN, VEEI, VAPS, VAPT, etc...) soient très bien conservés. En revanche, les séquences répétées du bloc 1 sont parfaitement conservées entre les deux isolats. Aussi compte tenu de la variabilité intrinsèque de ce bloc 2 d'un isolat à l'autre, la définition 70 % d'homologie s'entend pour l'antigène LSA-3 des différents isolats à l'exclusion des séquences répétées du bloc 2.

L'invention porte également sur les anticorps polyclonaux ou monoclonaux qui reconnaissent spécifiquement les molécules polypeptidiques de l'invention.

Ces molécules de l'invention peuvent être utilisées pour la mise en oeuvre de méthodes de diagnostic et la fabrication de trousses permettant de détecter l'existence de l'infection par P.falciparum ; cette méthode peut être soit un dosage d'anticorps spécifiques circulant par la mise en oeuvre de méthodes sérologiques classiques par mise en contact d'un des antigènes ci-dessus avec un fluide biologique de l'individu en question, soit des méthodes de dosages d'antigènes en utilisant des anticorps polyclonaux ou monoclonaux obtenus par des méthodes classiques d'obtention de tels anticorps par les antigènes correspondants. Dans les coffrets ou kits des diagnostics de l'invention, les réactifs permettant la détection des complexes antigènes/anticorps produits, pouvant également porter un marqueur ou être susceptibles d'être reconnus à leur tour par un réactif marqué sont présents. Selon que l'on souhaite réaliser un test antigène ou un test sérologique, le kit comprend soit les anticorps soit les antigènes de l'invention.

L'invention porte également sur toutes les séquences de nucléotides codant pour un polypeptide de l'invention ainsi que sur tout acide nucléique recombinant contenant au moins une séquence de nucléotides de l'invention, insérée dans un acide nucléique hétérologue vis à vis de ladite séquence de nucléotides.

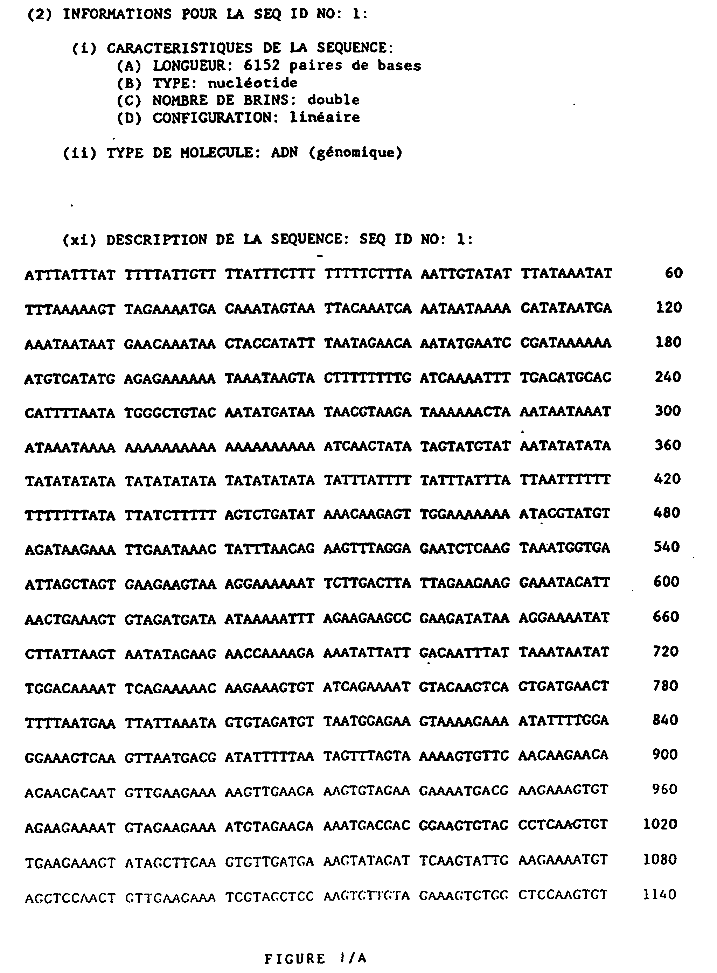

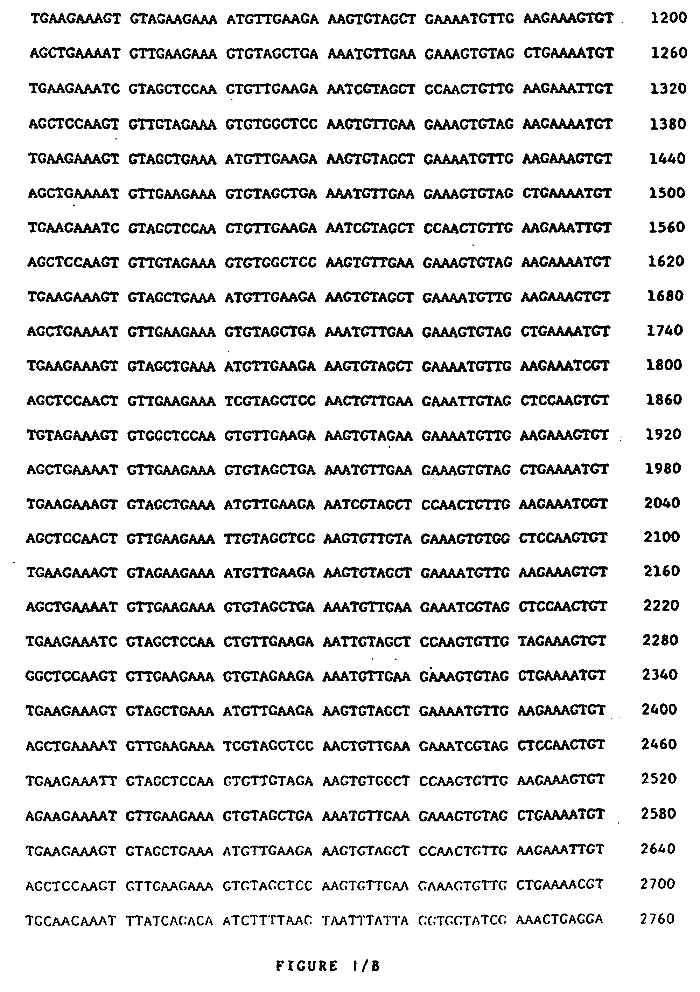

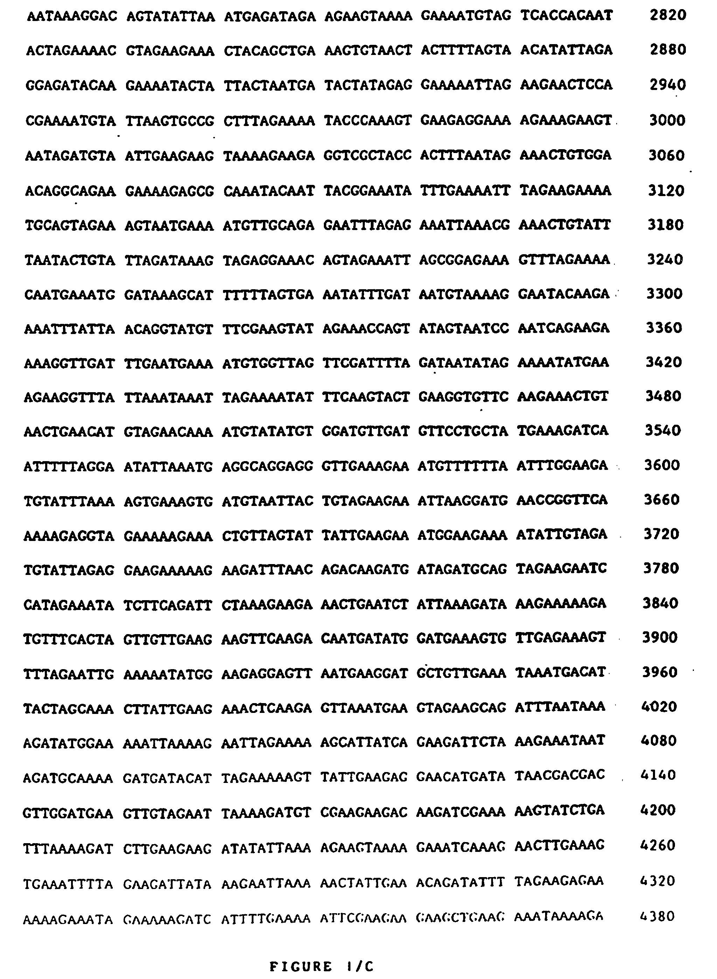

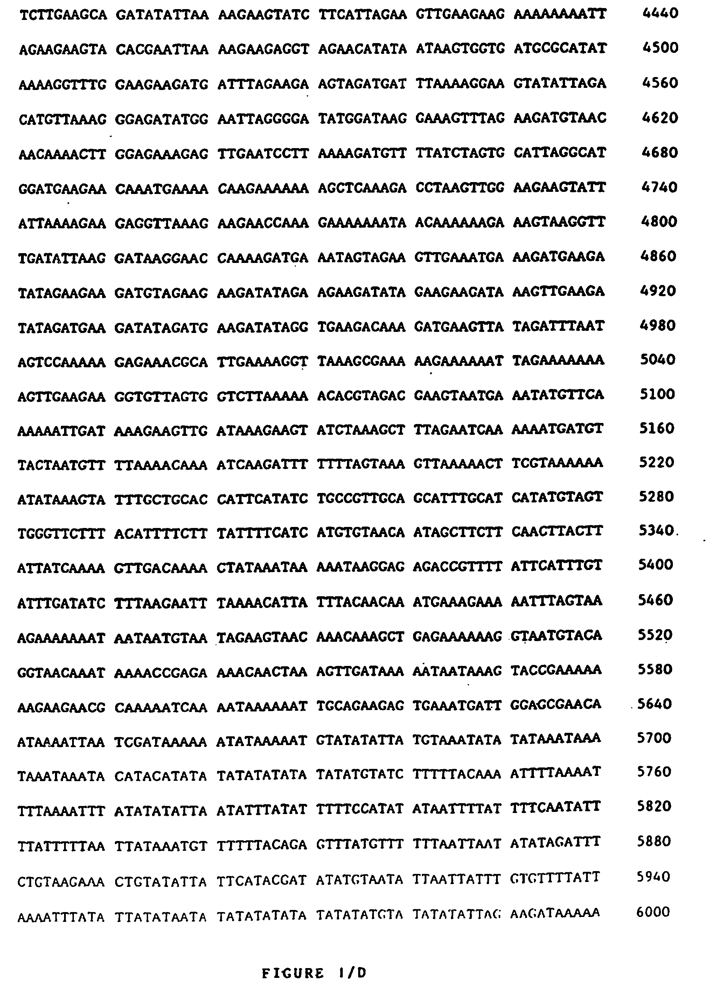

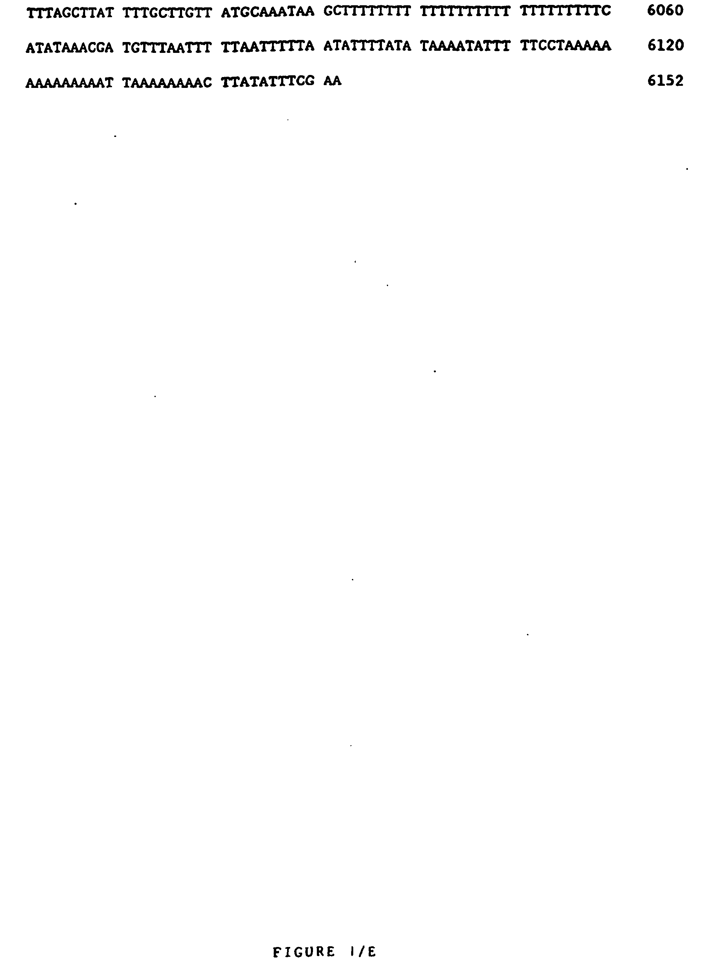

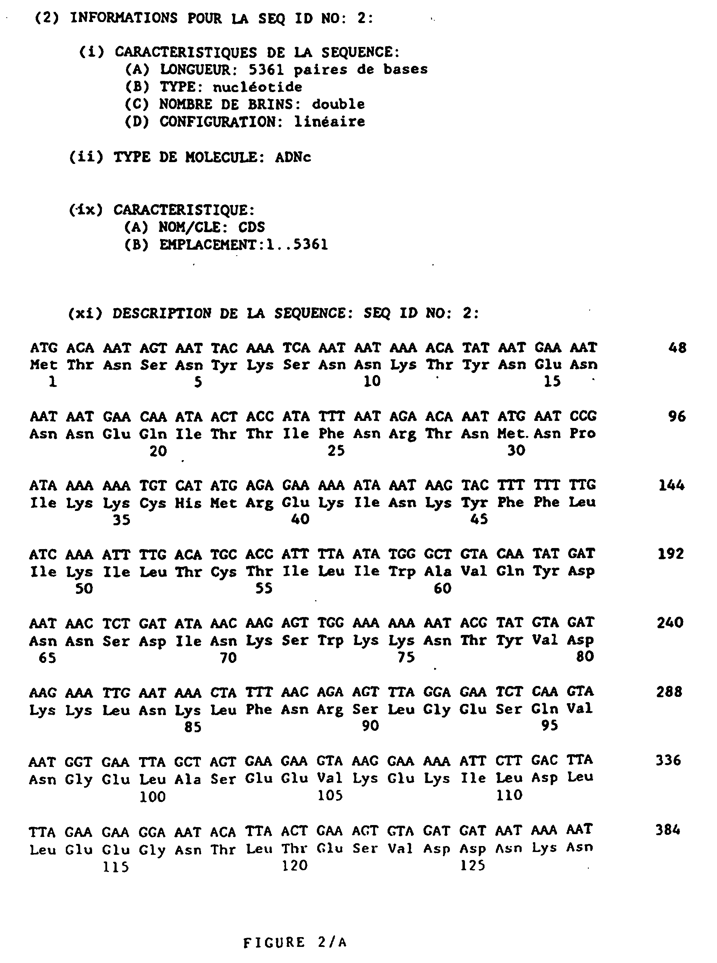

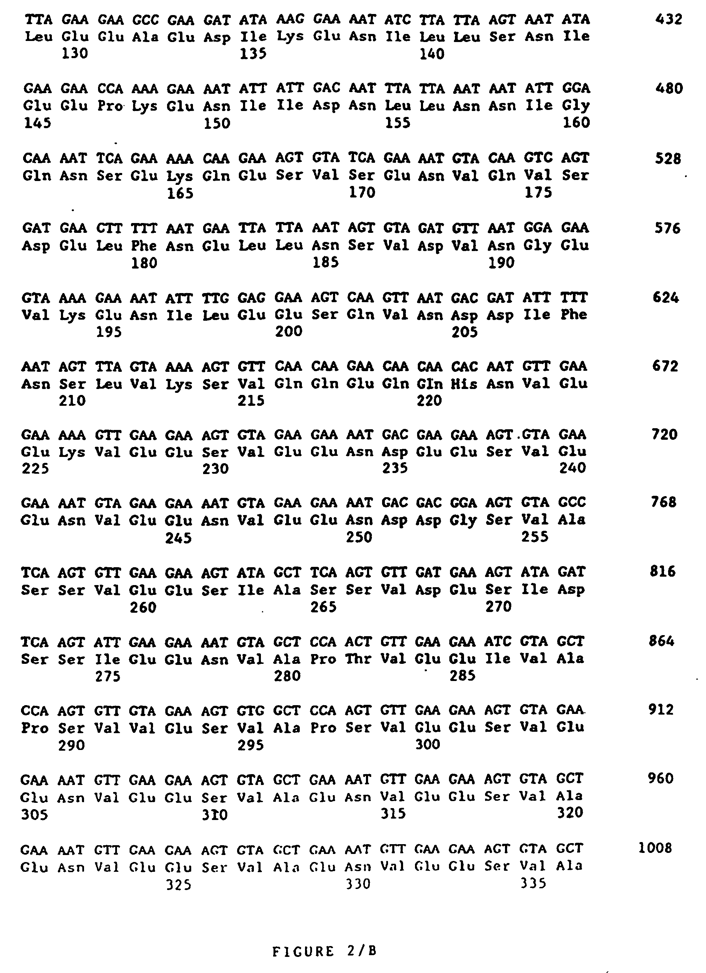

Font partie de l'invention les séquences d'acides nucléiques codant pour LSA-3 ou ses fragments immunogènes et répondant à l'une des définitions suivantes :
(a) l'enchaînement de nucléotides tel que représenté dans SEQ ID n° 1 de la figure 1, ou
(b) l'enchaînement de nucléotides représenté dans SEQ ID n° 2 de la figure 2,
(c) un enchaînement présentant au moins 70% d'homologie avec celui de la figure 1, ou de la figure 2 ou,
(d) un enchaînement de nucléotides complémentaires à ceux présentés en (a), (b) ou (c).

Par codant pour LSA-3, on entend tant le gène représenté dans SEQ ID n° 1 de la figure 1 que le cDNA représenté dans SEQ ID n° 2 de la figure 2.

L'invention concerne plus particulièrement un acide nucléique recombinant, dans lequel la séquence de nucléotides de l'invention est précédée d'un promoteur (notamment un promoteur inductible) sous le contrôle duquel la transcription de ladite séquence est susceptible d'être effectuée et, le cas échéant, suivie d'une séquence codant pour des signaux de terminaison de la transcription.

L'invention porte également sur la séquence codante issue du clone T9/96, représenté sur la figure 3 par la SEQ ID n° 3.

Dans cette séquence, le fragment CT1 est compris entre les nucléotides 67 et 126, le fragment 679 commence au nucléotide 206 et le fragment 729 RE est compris entre les nucléotides 547 et 630.

L'invention porte enfin sur tout vecteur recombinant, utilisé en particulier pour le clonage d'une séquence nucléotidique de l'invention, et/ou pour l'expression du polypeptide codé par cette séquence, et caractérisé en ce qu'il contient un acide nucléique recombinant, tel que défini ci-dessus, en l'un de ses sites non essentiel pour sa réplication.

A titre d'exemple de vecteur sus-mentionné, on citera les plasmides, les cosmides, les phages ou les virus.

A ce titre, l'invention concerne plus particulièrement le plasmide pK 1.2. déposé à la CNCM sous le n°I-1573.

L'invention a également pour objet un procédé de préparation d'un polypeptide de l'invention, par transformation d'un hôte cellulaire à l'aide d'un vecteur recombinant de type sus-indiqué, suivie de la mise en culture de l'hôte cellulaire ainsi transformé, et de la récupération du polypeptide dans le milieu de culture.

Ainsi, l'invention concerne tout hôte cellulaire transformé par un vecteur recombinant tel que défini ci-dessus, et comprenant les éléments de régulation permettant l'expression de la séquence de nucléotides codant pour un polypeptide selon l'invention.

L'invention porte de même sur des amorces d'ADN (ou d'ARN) utilisables dans le cadre de la synthèse de séquences nucléotidiques et/ou polypeptidiques de l'invention, par la technique du PCR (Polymerase Chain Reaction) ou toute autre méthode aujourd'hui connue pour amplifier les acides nucléiques telles la LCR, CPR, ERA, SPA, NASBA, etc...

L'invention concerne toute amorce d'ADN ou d'ARN, caractérisée en ce qu'elle est constituée d'environ 10 à 25 nucléotides, identiques ou complémentaires aux 10 à 25 premiers nucléotides de la séquence de nucléotides codant pour une séquence peptidique selon l'invention ou identiques aux 10 à 25 derniers nucléotides de ladite séquence.

Ainsi la présente invention porte également sur un procédé de préparation d'un polypeptide de l'invention comprenant les étapes suivantes :
- le cas échéant, l'amplification préalable par des techniques classiques de la quantité de séquences de nucléotides codant pour ledit polypeptide à l'aide de deux amorces d'ADN choisies de manière appropriée,
- la mise en culture, dans un milieu de culture approprié, d'un hôte cellulaire préalablement transformé par un vecteur contenant un acide nucléique selon l'invention comprenant la séquence nucléotidique codant pour ledit polypeptide, et
- la récupération, à partir du susdit milieu de culture du polypeptide produit par ledit hôte cellulaire transformé.

A titre d'exemple d'amorces d'ADN ou d'ARN selon l'invention, on citera les couples de séquences suivants :
S1 :
S2:
dont les positions respectives sur le gène LSA-3/K1 représenté sur la figure 1 sont de 695 à 722 et de 829 à 799 (en lecture inverse), ou le couple :
6.1 :
6.2 :
dont les positions respectives sont 2668 à 2692 pour 6.1. et 3456 à 3433 pour 6.2. (lecture inverse).

Les informations sur les séquences ID n° 4, ID n° 5, ID n° 6 et ID n° 7 sont détaillées à la fin de la description.

Les peptides de l'invention peuvent également être préparés par les techniques classiques de la synthèse de peptides. Cette synthèse peut être réalisée en solution homogène ou en phase solide. Par exemple, on aura recours à la technique de synthèse en solution homogène décrit par HOUBENWEYL dans l'ouvrage intitulé « Meuthode der Organischen Chemie » (Méthode de la Chimie Organique édité par E. Wunsch, vol. 15-I et II. THIEME, Stuttgart 1974 ou celle décrite par R..D. MERRIFIELD dans l'article intitulé « Solid phase peptide synthesis » (J. Am. Chem. Soc., 45, 2149-2154).

L'invention porte également sur les oligomères hydrosolubles des peptides monomères sus-indiqués.

L'oligomérisation peut provoquer un accroissement de l'immunogénicité des peptides monomères selon l'invention. Sans qu'une telle indication chiffrée puisse être considérée comme limitative, on mentionnera néanmoins que ces oligomères peuvent, par exemple, contenir de 2 à 10 unités monomères.

On peut avoir recours, pour réaliser l'oligomérisation, à toute technique de polymérisation couramment utilisée dans le domaine des peptides, cette polymérisation étant conduite jusqu'à l'obtention d'un oligomère ou polymère contenant le nombre de motifs monomères requis pour l'acquisition de l'immunogénicité désirée.

Une méthode d'oligomérisation ou de polymérisation du monomère consiste dans la réaction de celui-ci avec un agent de réticulation tel que le glutaraldéhyde.

On peut également avoir recours à d'autres méthodes d'oligomérisation ou de couplage, par exemple à celle mettant en jeu des couplages successifs d'unités monomères, par l'intermédiaire de leurs fonctions terminales carboxyle et amine en présence d'agents de couplage homo- ou hétéro-bifonctionnels.

L'invention concerne encore les conjugués obtenus par couplage covalent des peptides selon l'invention (ou des susdits oligomères) à des molécules porteuses permettant notamment d'augmenter l'immunogénicité (naturelles ou synthétiques), physiologiquement acceptables et non toxiques, par l'intermédiaire de groupements réactifs complémentaires respectivement portés par la molécule porteuse et le peptide. A titre d'exemple de molécules porteuses ou supports macromoléculaires entrant dans la constitution des conjugués selon l'invention, on mentionnera des protéines naturelles, telles que l'anatoxine tétanique, l'ovalbumine, des sérums albumines, des hémocyamines, le PPD de la tuberculine (PPD : « Purifled Protein Derivative »), etc...

A titre de supports macromoléculaires synthétiques, on mentionnera par exemple des polylysines ou des poly(D-L-alanine)-poly(L-lysine).

A titre de supports hydrocarbones ou lipidiques, on mentionnera les acides gras, saturés ou insaturés, et préférentiellement ceux en C16 ou C18 de type oleyl ou palmitoleyl.

Enfin et sans être limitatifs, les antigènes ou peptides selon l'invention peuvent être couplés à des supports classiques ou adsorbées sur de tels supports notamment des microsphères ou billes de latex ou de polystyrène, ou incorporés dans des particules Ty1.

Pour synthétiser les conjugués selon l'invention, on peut avoir recours à des procédés connus en soi, tels que celui décrit par Frantz et Robertson dans Infect. and Immunity, 33, 193-198 (1981), ou celui décrit dans Applied and Environmental Microbiology, (octobre 1981), vol. 42, n° 4, 611-614 par P.E. Kauffman en utilisant le peptide et la molécule porteuse appropriée.

Les acides nucléiques de l'invention peuvent être préparés soit par un procédé chimique, soit par d'autres procédés.

Un mode de préparation approprié des acides nucléiques comportant au maximum 200 nucléotides (ou 200 pb, lorsqu'il s'agit d'acides nucléiques bicaténaires) de l'invention comprend les étapes suivantes :
- la synthèse d'ADN en utilisant la méthode automatisée des β-cyanethylphosphoramidite décrite dans Bioorganic Chemistry 4; 274-325 (1986),
- le clonage des acides nucléiques ainsi obtenus dans un vecteur approprié et la récupération de l'acide nucléique par hybridation avec une sonde appropriée.

Un mode de préparation, par voie chimique, d'acides nucléiques de longueur supérieure à 200 nucléotides a déjà été été décrit dans W0 92/13884.

L'invention concerne également des trousses de diagnostic qui comportent une ou plusieurs amorces d'amplification spécifiques du gène LSA-3 et permettant de détecter la présence du gène ou du mRNA chez un individu susceptible d'être infecté par P.falciparum.

L'invention porte aussi sur des compositions pharmaceutiques ou vaccinales dans lesquelles l'un au moins des produits selon l'invention se trouve associé à des excipients pharmaceutiquement acceptables, solides ou liquides, adaptés à la construction de formes orales, oculaires ou nasales, ou des excipients adaptés à la construction des formes d'administration rectale, ou encore avec des excipients gétatineux pour l'administration vaginale. Elle concerne aussi des compositions liquides isotoniques contenant l'un au moins des conjugués selon l'invention, adaptées à l'administration sur les muqueuses, notamment oculaires ou nasales ou pulmonaires.

Avantageusement, les compositions vaccinales selon l'invention contiennent en outre un véhicule, tel que la polyvinyl-pyrrolidone, facilitant l'administration du vaccin. A la place de la polyvinyl-pyrrolidone, on peut utiliser tout autre type d'adjuvant au sens classique que l'on donnait autrefois à cette expression, c'est-à-dire d'une substance permettant l'absorption plus aisée d'un médicament ou facilitant son action dans l'organisme. A titre d'exemples d'autres adjuvants de ce dernier type, on mentionnera encore la carboxyméthyl-cellulose, les hydroxydes et phosphates d'aluminium, la saponine ou tous autres adjuvants de ce type, bien connus de l'homme de l'art. Enfin, elles contiennent si besoin un adjuvant immunologique, notamment du type muramylpeptide.

L'invention concerne aussi des compositions pharmaceutiques contenant à titre de substance active l'un au moins des anticorps polyclonaux ou monoclonaux définis précédemment en association avec un véhicule pharmaceutiquement acceptable.

L'invention porte enfin sur une méthode d'immunisation d'un individu susceptible d'être infecté par P. falciparum par injection d'une molécule peptidique ou d'un oligomère tel que décrit précédemment, seul ou en association avec d'autres types de molécules susceptibles de protéger ledit individu contre une infection ultérieure, la molécule polypeptidique ou antigénique ou les lipopeptides naturels ou recombinants sont, soit utilisés seuls, soit adsorbés ou couplés à des microsphères ou billes de latex ou de polystyrène.

Des caractéristiques supplémentaires de l'invention apparaîtront encore dans les exemples illustrés des figures qui suivent et montrent les caractéristiques particulières des molécules de l'invention par rapport à d'autres antigènes du stade pré-érythrocytaire du parasite.
La figure 1 représente la séquence ID n° 1 d'ADN génomique de 6152 paires de bases du gène LSA-3, elle est issue du clone K1.2 lui-même issu d'un isolat thaïlandais.
La figure 2 représente la séquence ID n° 2 du cDNA et la séquence polypeptidique de l'antigène LSA-3. La séquence de DNA représente 5361 paires de bases.
La figure 3 représente la séquence ID n° 3 de la partie séquencée dans le clone parasitaire T9/96 (1890 paires de bases), la ligne du haut étant la séquence nucléotidique et la ligne du bas la séquence peptidique. Dans ce clone, la séquence CT1 est comprise entre les nucléotides 67 et 126, le fragment DG 679 proprement dit commençant au nucléotide 207. Le fragment 729 RE est compris entre les nucléotides 547 et 629.
La figure 4a représente schématiquement les positionnements relatifs des séquences répétées et non répétées, des introns et des exons dans les souches K1 et T9/96, les clones 679 et 729 étant issus de ce dernier.
La figure 4b représente le HCP (Hydrophobic Cluster Plot) de la séquence peptidique du clone DG729.
La figure 5 représente les quantités d'immunoglobulines produites dans le sérum de chimpanzé Nuria avant et après l'immunisation par différents peptides de LSA-3.
La figure 6 indique le titre en anticorps spécifique de différentes espèces de souris immunisées soit avec un peptide soit avec un lipopeptide correspondant.
La figure 7 montre l'inhibition de l'invasion des cellules hépatiques par les sporozoïtes par des sérums hyper-immuns obtenus après immunisation par différents peptides immuno-purifiés contre du LSA-3 entier.
La figure 8 représente la comparaison d'un antigène issu de LSA-3 avec deux autres antigènes sur l'immunité de type T.

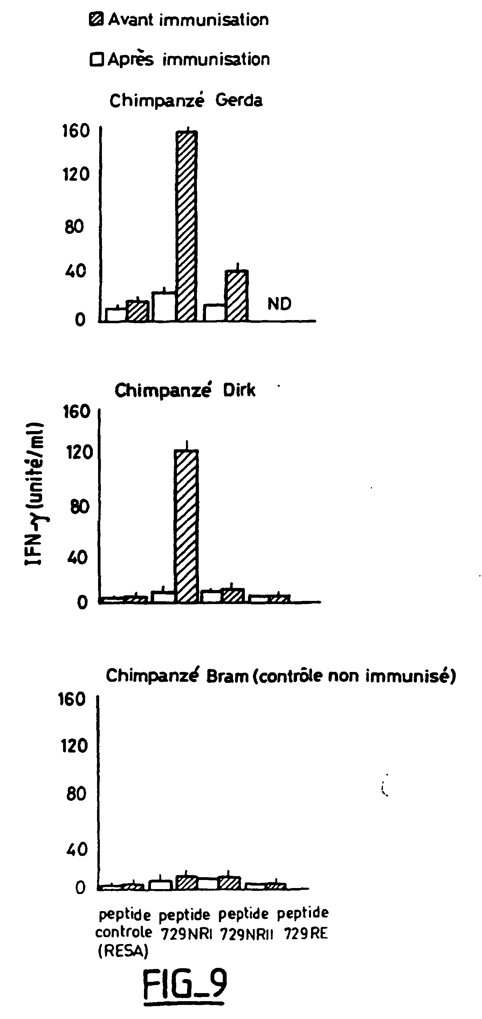

La figure 9 représente l'induction de l'interféron-γ chez les chimpanzés Gerda et Dirk par les peptides issus de la molécule LSA-3.
La figure 10 représente les résultats de lymphoprolifération des PBMC d'un individu protégé par injection de sporozoïtes irradiés contre des peptides issus des antigènes LSA-1 et LSA-3.

### Exemple 1 : Clonage et séquençage du gène LSA-3

### 1 ) séquençage

Le criblage initial de la génothèque provenant du clone parasitaire T9/96 avec le sérum d'un missionnaire soigné de façon continue en prophylaxie nous a permis d'isoler 120 clones correspondant à des molécules exprimées au stade sporozoïque et/ou hépatique du cycle de P.falciparum. Le clone 729S a été utilisé comme sonde pour cribler une banque génomique de la souche K1 thaïlandaise déjà citée plus haut qui contient de grands fragment Eco R1 clonés dans le phage lambda gt10. Un insert de 6,85 kilobases contenant le gène entier a été purifié de cette génothèque et recloné dans un plasmide pUC18 pour séquençage et caractérisation. Chez P.falciparum dont le génome est très riche en base A :T (80%), cette approche est souvent rendue difficile par la rareté des sites de restriction utilisables et par l'instabilité ou même l'impossibilité de cloner certains fragments lorsqu'ils sont insérés en vecteurs plasmidiques.

La structure du gène est représentée dans la figure 4 et présente les caractéristiques suivantes :
a) un mini exon 1 codant à son extrémité 3' pour un peptide signal hydrophobe ;
b) un court intron (168 paires de bases) inclus entre des sites consensus de donneurs et accepteurs de splicing ;
c) un deuxième exon de cinq kilobases qui code pour une région organisée de 1,8 kilobases et composé d'un arrangement de 7 blocs de 4 amino acides, et d'une région hydrophobe en 3' qui pourrait correspondre à un ancrage du type glycosyl-phosphatidyl-inositol (GPI).

Une investigation détaillée du polymorphisme du LSA-3 a été réalisée en séquençant le clone 679 qui contient l'essentiel des séquences répétées du gène LSA-3 et une portion de 1 kilobase de la fraction non répétée en 3', la séquence de ce fragment étant représentée dans la figure 3 entre les nucléotides 207 et 1890.

Les répétitions de la souche K1 sont les suivantes :
Bloc 1 : (aa223) VEEK VEES VEEN DEES VEEN VEEN VEEN DDGS VASS VEES IASS VDES IDSS IEEN (aa278)
Bloc 2 : (aa279) VAPT VEEIVAPS WESVAPS VEESVEEN
   VEESVAEN VEESVAEN VEESVAEN VEESVAEN
   VEEIVAPT VEEIVAPT VEEIVAPS WESVAPS VEESVEEN
   VEESVAEN VEESVAEN VEESVAEN VEESVAEN VEESVAEN
   VEEIVAPT VEEIVAPT VEEIVAPS WESVAPS VEESVEEN
   VEESVAEN VEESVAEN VEESVAEN VEESVAEN VEESVAEN
   VEESVAEN VEESVAEN
   VEEIVAPT VEEIVAPT VEEIVAPS WESVAPS VEESVEEN
   VEESVAEN VEESVAEN VEESVAEN VEESVAEN
   VEEIVAPT VEEIVAPT VEEIVAPS WESVAPS VEESVEEN
   VEESVAEN VEESVAEN VEESVAEN
   VEEIVAPT VEEIVAPT VEEIVAPS WESVAPS VEESVEEN
   VEESVAEN VEESVAEN VEESVAEN VEESVAEN
   VEEIVAPT VEEIVAPT VEEIVAPS WESVAPS VEESVEEN
   VEESVAEN VEESVAEN VEESVAEN
   VEESVAPT VEEIVAPS VEESVAPS VEESVAEN (aa818)
Bloc 3 :
   (aa1537)DEDI EEDV EEDI EEDI EEDK VEDI DEDI DEDI GEDK DEVI (aa1576)

Les répétitions dans le clone T9/96 telles que déterminées dans la demande de brevet n° FR 9101286 du 05 février 1991, sont les suivantes :
Bloc 1 : VEEK VEES VEEN DEES VEEN VEEN VEEN DDGS VASS VEES IASS VDES IDSS IEEN
Bloc 2 : VAPT VEEIVAPT VEEIVAPS WESVAPS VEESVAPS
   VEESVAEN VEESVAEN
   VEEIVAPS
   VEESVAEN VEESVAEN VEESVAEN VEESVAEN VEESVAEN
   VEEIVAPT VEESVAPT VEEIVAPT VEESVAPT VEEIWPS VEESVAPS
   VEESVAEN VEESVAEN VEESVAEN VEESVAEN VEESVAEN
   VEEIVAPS VEEIVAPT
   VEESVAEN

L'exon 2 du gène LSA3 comporte 2 régions répétées pouvant être décomposées en 3 blocs comme indiqué sur la figure 4 :
- le bloc 1, codant pour un enchaînement de 14 tétrapeptides. Ce bloc est conservé à 100% en acides aminés et en acides nucléiques entre T9/96 et K1. Seuls les tétrapeptides VEES et VEEN sont retrouvés dans le bloc 2.
- le bloc 2 code, dans K1, pour 127 tétrapeptides correspondant à l'enchaînement de différents octapeptides, eux-mêmes formés par combinaison de 2 des 7 tétrapeptides ou motifs de base (VEES, WES, VEEN, VEEI, VAEN, VAPS, VAPT). Le nombre de répétitions et l'arrangement de ces octapeptides varient selon les motifs et semblent être spécifiques du clone K1.2. En effet, dans le clone T9/96, le bloc 2 (53 tétrapeptides) correspond également à l'enchaînement d'octapeptides formés à partir des 7 mêmes tétrapeptides de base (et d'un 8ème motif VVPS n'existant pas dans K1), mais avec un nombre et un arrangement différents de ces répétitions.
- le bloc 3 est constitué de l'enchaînement de 10 tétrapeptides dégénérés et différents de ceux des blocs 1 et 2. Ce bloc a été séquencé uniquement dans la souche K1. Cependant, des résultats préliminaires obtenus par PCR avec le clone T9/96 et plusieurs autres souches de laboratoire indiquent qu'il n'existe pas de polymorphisme de taille dans cette région.

Les régions non-répétées de l'exon 2 sont particulièrement bien conservées. En effet, la comparaison de séquence entre T9/96 et K1 a pu être faite sur 315 bp en 5' du bloc 1 et sur 763 bp en 3' du bloc 2. L'homologie est de 99,4% en acides nucléiques et 98,6% en acides aminés.

La comparaison des séquences du clone 679 issu du clone de P.falciparum T9/96, et de la séquence correspondante du LSA-3 issue de l'isolat K1 montre que le gène est bien conservé, les différences les plus notables étant observées dans la région répétée où les blocs de 4 amino acides sont bien conservés mais varient dans leur nombre et leur organisation.

Au contraire les parties 5' et 3' non répétées apparaissent comme particulièrement bien conservées montrant jusqu'à 100% d'homologie dans la région 5' où des épitopes B et T ont déjà été identifiés.

Des amplifications d'ADN, notamment par PCR de différentes souches de P.falciparum avec 8 paires d'amorces réparties sur l'ensemble du gène LSA-3 ont montré que, excepté avec celles entourant les régions répétées, tout le génome donne des produits PCR de taille similaire ce qui suggère que l'antigène LSA-3 est bien conservé.

Diverse sondes LSA-3, choisies dans les régions répétées et non répétées, ont été hybridées à faible stringence avec les ADN de différentes espèces de Plasmodium et n'ont permis d'identifier aucun gène homologue à LSA-3, sauf chez le parasite de chimpanzé P.reichenowi, ce qui confirme la proche parenté de cette espèce avec P.falciparum.

De façon surprenante, l'antigène analogue à LSA-3 trouvé dans P. yoelii, qui donne clairement des réactions immunologiques croisées à la surface du sporozoïte avec des anticorps contre le fragment 729S, ne semble pas être conservé au niveau de la séquence nucléotidique. Enfin la comparaison des séquences LSA-3 avec les bases de données n'a révélé aucune homologie avec des molécules connues, à l'exception de la région répétée dont certains motifs présentent une forte analogie avec les répétitions d'un gène de Staphilococus xylosis, mais aussi avec deux antigènes de P.falciparum, RESA et Pf11.1, tous deux exprimés au cours du stade sanguin du parasite. Cette homologie est essentiellement due à la richesse en séquences « glu-glu » de ces antigènes et des répétitions de LSA-3.

### 2) Clonage :

L'insert DG729 et d'autres régions de l'exon 2 de la souche K1 ont été clonés dans un vecteur d'expression procaryotique pGEX, vecteur commercialisé par la société In Vitrogene Corp (San Diego USA). Ce vecteur produit une protéine de fusion avec la glutathion -S-transferase (GST) de Schistosoma mansoni et permet une purification facile des protéines recombinantes par affinité du billes de glutathion-agarose. Les peptides d'expression à partir de ces vecteurs sont nommés :
- pour la protéine LSA-3 entière : protéine REC,
- ou pour le fragment 729S : 729PGEX.

Les tentatives de clonage d'autres fragments notamment le fragment 1-5 3NSREP, 3NFREP, 5NR, et 5SNREP ont posé des difficultés concernant soit le clonage soit la production et la purification des protéines en quantité suffisante pour des expériences d'immunisation.

Seuls les fragments 729, NN et 3PC ont permis de produire et de purifier des polypeptides recombinants correspondants en quantité suffisante pour l'analyse de l'antigénicité de la molécule.

### Exemple 2: Protection de chimpanzés immunisés contre des injections d'épreuve à faible ou forte dose :

2.1. Un chimpanzé Dirk préalablement immunisé par une fraction de LSA-3 en combinaison avec un autre antigène du même stade de développement du parasite, et présentant les effets décrits plus haut au point a), a été re-immunisé quelques années plus tard par des peptides et des protéines recombinantes correspondant à la même combinaison d'antigènes. De nouveau, ce chimpanzé s'avère protégé contre une infection d'épreuve à faible dose (2.10⁴ sporozoïtes) puis une infection d'épreuve à forte dose (5.10⁶ sporozoïtes). Comme au cours du premier challenge, on observe une réduction importante du nombre de schizontes détectés dans le foie après le challenge à forte dose ainsi qu'un infiltrat lympho-monocytaire autour des rares schizontes détectables (témoin d'une défense locale).
2.2. Protection partielle du chimpanzé Gerda : un autre chimpanzé a été immunisé uniquement par l'antigène LSA-3 (animal décrit dans les exemples 7 et 8 ci-après), à savoir le lipopeptide NR2 puis les protéines recombinantes (GST-729, GST-NN, GST-3PC) qui couvrent à elles trois 95 % de la molécule LSA-3 et adsorbées sur microsphères de latex. Cet animal s'avère être partiellement protégé contre une infection d'épreuve à forte dose (8.10⁶ sporozoïtes) puisqu'il présente une très faible parasitémie sanguine et une réduction de 90 % du nombre de schizontes hépatiques par rapport au contrôle suite à l'infection d'épreuve.
2.3 Protection partielle du chimpanzé Nuria : un chimpanzé immunisé par une fraction de l'antigène LSA-3 seul, à savoir une combinaison de peptides, de lipopeptides puis de protéines recombinantes correspondant à 95 % de la molécule LSA-3 et émulsifiées dans le Montanide ISA-51 (SEPPIC, 75 Quai d'Orsay, France), s'avère être partiellement protégé contre une infection d'épreuve à dose moyenne (1.10⁵ sporozoïtes). En effet, cet animal présente un retard significatif de l'apparition des parasites dans le sang par rapport à 4 témoins (chimpanzés immunisés par les antigènes pré-érythrocytaires LSA-1, SALSA ou STARP, et 1 animal témoin non immunisé), une parasitémie sanguine maxima plus faible et une chute de parasitémie plus rapide (24 heures au lieu de 3 jours), résultats qui traduisent une forte réduction du nombre de formes hépatiques induites chez cet animal par l'infection d'épreuve et en accord avec les résultats obtenus chez Gerda. Dans ce cas, l'examen des formes hépatiques n'a pas été réalisé.
2.4 Immunogénicité B. T chez les chimpanzés Demi, Karlien et Iris :Trois chimpanzés immunisés par les peptides LSA-3-NR1 et -RE et les lipopeptides -NR2 et -CT1, ainsi que par des peptides correspondant, pour chaque animal, à un autre antigène pré-érythrocytaire (LSA-1, SALSA ou STARP) présentent tous les 3 :
   - des réponses humorales élevées contre les épitopes B présents sur les peptides NR1, NR2 et RE. Les anticorps reconnaissent non seulement les peptides et les recombinants mais sont aussi fortement positifs sur les molécules natives du parasite, ce qui est apprécié par immunofluorescence sur les sporozoïtes et les stades hépatiques de Plasmosdium falciparum (mais négatifs vis-à-vis des stades érythrocytaires) ;
   - des réponses lymphoprolifératives élevées et spécifiques contre les 4 peptides LSA-3 ainsi que les épitopes T natifs présents à la surface des sporozoïtes de Plasmodium falciparum et de Plasmodium yoelii, chez lequel LSA-3 possède un homologue (non encore caractérisé).
   Les réponses B et T vis-à-vis des antigènes natifs sont un point important car:
   a) elles prouvent la bonne représentativité des molécules synthétiques ;
   b) elles signifient qu'au moment de l'infection, il y a de bonnes chances d'obtenir une réponse secondaire anamnestique ; c'est de fait ce qui a été observé chez le chimpanzé Nuria au moment du challenge. L'importance de cette observation est confortée par le fait que la même réponse secondaire n'a pas été obtenue vis-à-vis des autres antigènes tels que LSA-1 et STARP.
2.5. Immunogénicité chez l'Aotus : un singe-hibou (Aotus trivirgatus) immunisé par les 2 peptides LSA-3-NR1 et -RE et les 2 lipopeptides -NR2 et -CT1, puis re-stimulé par les protéines recombinantes correspondant à 95 % de la molécule LSA-3 et adsorbées sur microsphères comme décrit ci-dessus, présente des réponses lymphoprolifératives élevées et spécifiques contre les épitopes T présents sur ces mêmes peptides.

Pour ce qui est de la réponse in vivo des différents chimpanzés ainsi pré-immunisés, les résultats soulignent l'excellente immunogénicité (B et T) de LSA-3, sous forme peptide, lipopeptide et recombinants, et dans tous les modèles animaux testés jusqu'à présent, soit 6/6 chimpanzés (outbred), 1/1 aotus, et chez toutes les souris immunisées (>20). Notons que les résultats des formulations lipopeptidiques (qui peuvent être utilisées chez l'Homme) ont été obtenus par injections sous-cutanées en l'absence de tout adjuvant.

### Exemple 3 : Identification d'épitope CTL

La méthode utilisée pour identifier les CTL est celle décrite dans Fidock et al, (1994), J. Immunol. 153 : 190 ou dans Bottius et al, (1996), J. Immunol 156 :2874-2884.

Des épitopes CTL (pour lymphocytes T cytotoxiques) ont été identifiés dans les peptides NR2, RE et CT1 grâce à des tests de cytotoxicité effectués à partir des PBMC des chimpanzés Dirk, Gerda, Nuria, Demi, Karlien et Iris décrits plus haut.

Chez l'homme, 8 épitopes CTL additionnels, dont 7 situés dans la région 3' non-répétée, ont pu être mis en évidence à partir des PBMC d'individus appartenant à 3 haplotypes différents (MHC classe 1-A2, -B8 et -B53) et vivant en région d'endémie (Gambie) (résultats non publiés). De plus, le séquençage des 2 épitopes CTL restreints par B53 a démontré une parfaite conservation de leurs séquences nucléotidique et peptidique dans plusieurs souches du Kenya et de Gambie.

Au total, nous avons identifié 11 épitopes CTL dans la molécule LSA-3, ce qui est considérable. Par ailleurs, 5 chimpanzés/6 ont développé des réponses CTL contre le peptide NR2 après immunisation par le lipopeptide NR2 sans adjuvant, ce qui est un résultat remarquable pour des animaux non-consanguins. D'autre part, dans la mesure où les anticorps développés par Nuria ne présentaient aucune activité inhibitrice de l'invasion des sporozoïtes de Plasmodium falciparum, on peut supposer que la protection observée dépendait des réponses cellulaires, en particulier des CTL.

### Exemple 4 : Comparaison des titres anticorps avant ou après immunisation par différents peptides

### 4.1. Comparaison des réponses en anticorps induites par différents peptides dans différents animaux immunisés.

La méthode utilisée est celle décrite dans Behr et al, (1992), J. Immunol 149 : 3321.

La réactivité est exprimée en ratio Elisa, c'est-à-dire la densité optique mesurée à 496 nanomètres du sérum après immunisation rapporté à la densité optique du même sérum avant immunisation. La première colonne indique l'animal immunisé, la deuxième colonne l'immunogène reçu par l'animal, la 3ème colonne indique le nombre d'injections réalisées ainsi que le support accompagnant le peptide injecté : RP signifie protéine recombinante, RP/B signifie protéine recombinante adsorbée sur billes de latex, P signifie peptide et LP lipopeptide. Il faut préciser en outre que les lipopeptides sont injectés dans du sérum physiologique, les peptides et les protéines recombinantes sont adsorbés sur les billes de latex ou dans une émulsion avec un adjuvant montanide ISA-51.

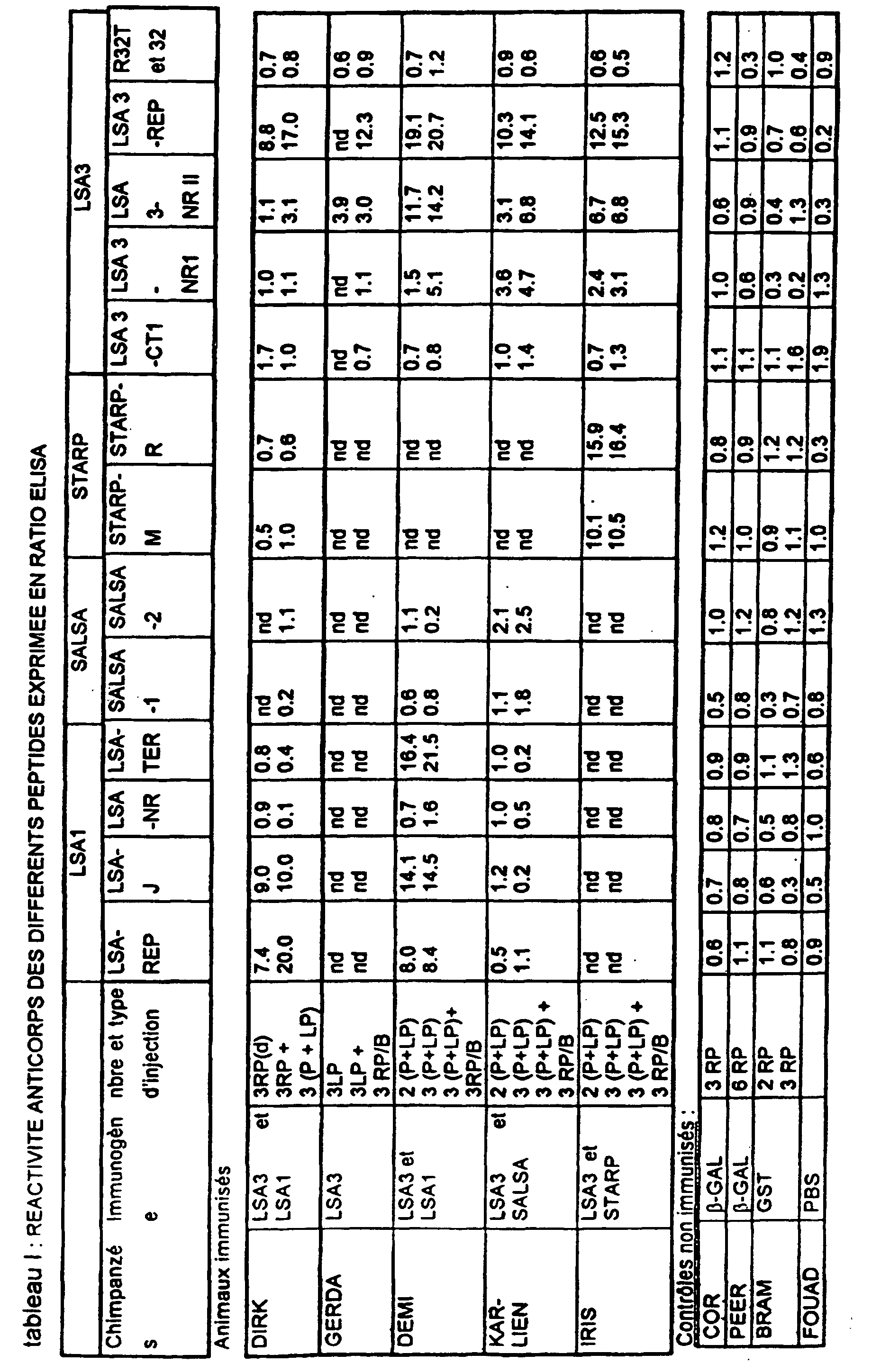

### 4.2. Titre des anticorps obtenus :

Le tableau 2 représente les titres d'anticorps des sérums obtenus chez les chimpanzés par immunofluorescence sur les antigènes natifs présents à la surface des différents stades (sporozoïques, hépatiques et sanguins) de P. falciparum, P. yoelii et P. berghei.

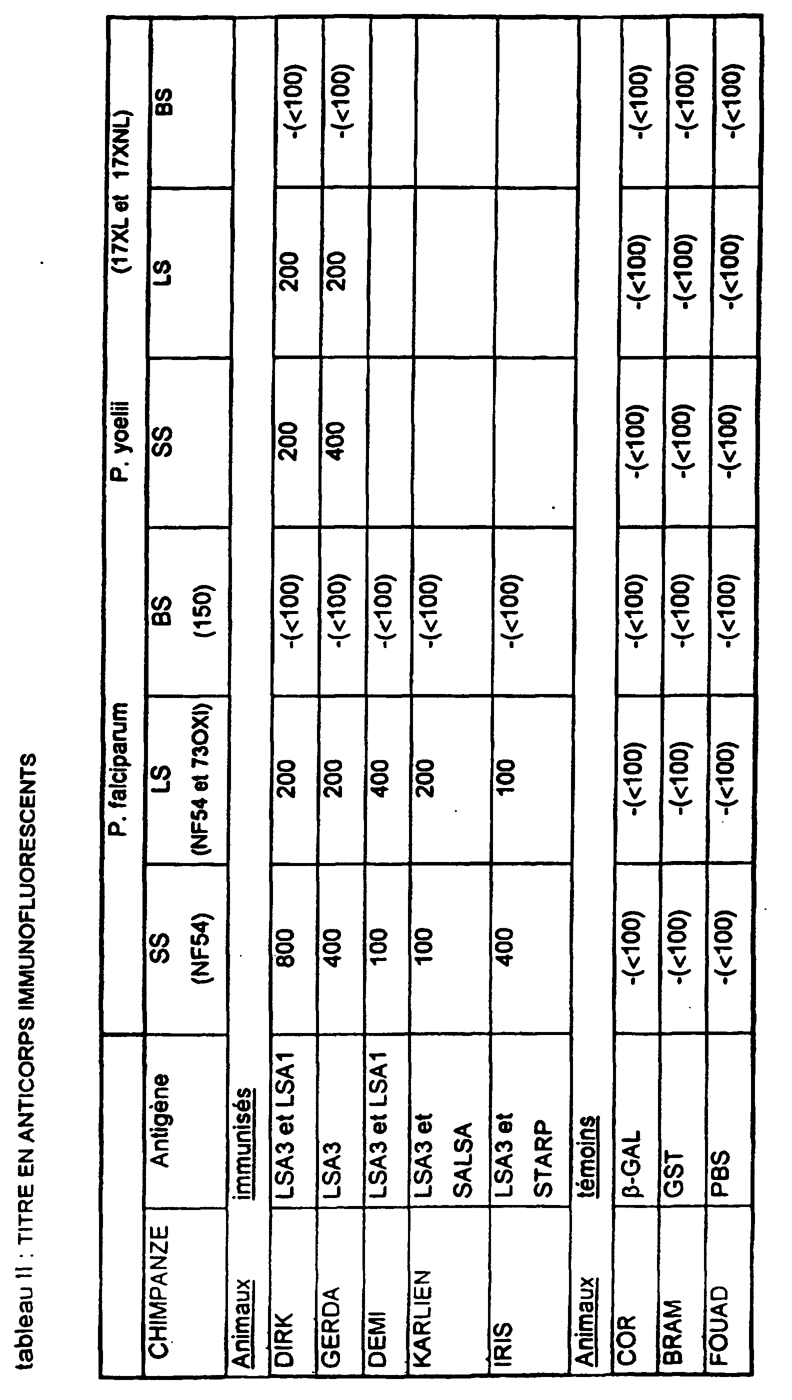

4.3 Réponse lymphoproliférative des PBMC des différents chimpanzés après stimulation in vitro soit par les différents peptides soit par les antigènes natifs présents à la surface des sporozoïtes. Cette réponse a été mesurée par incorporation de thymidine tritiée dans des PBMC (cellules de sang périphériques) soit après stimulation par les peptides LSA-3 (tableau III) ou après stimulation in vitro avec des sporozoïtes (tableau IV).

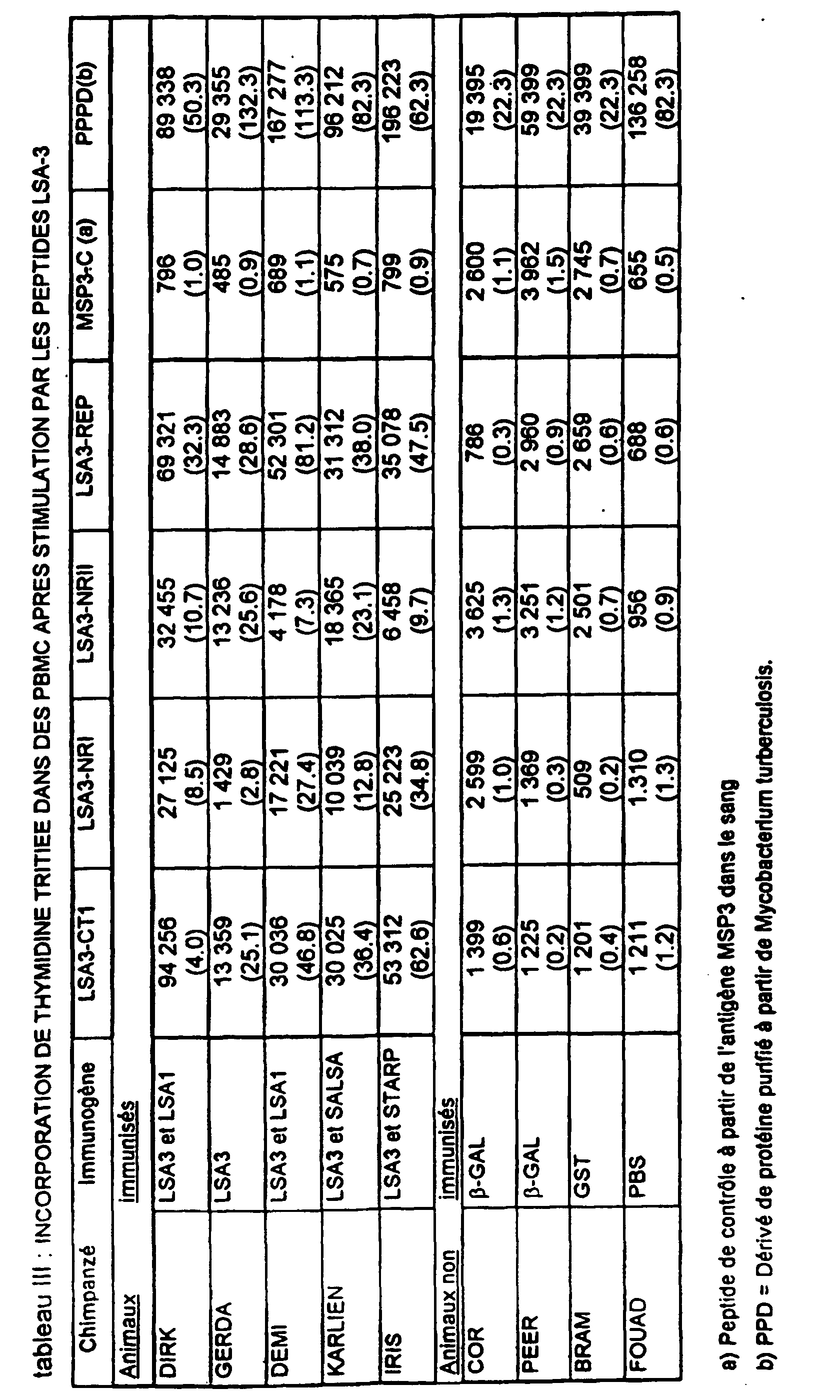

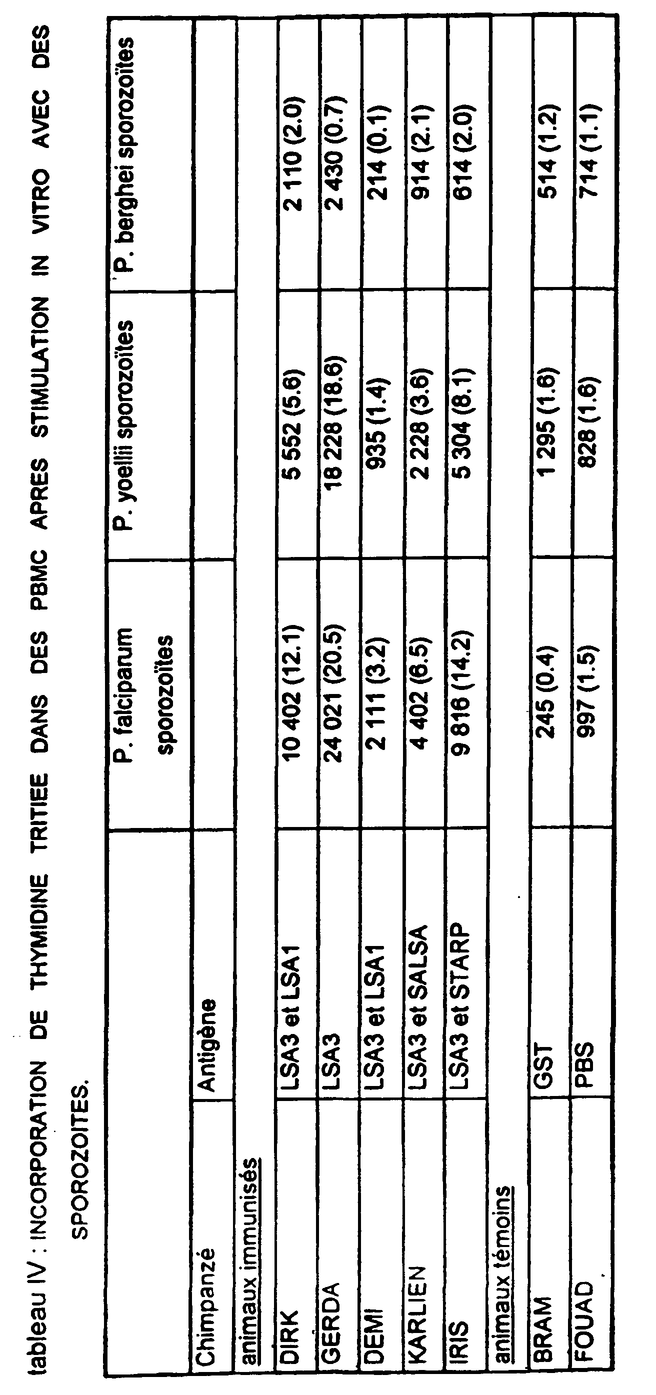

Les réponses lymphoprolifératives sont indiquées en différence de comptage en coups par minute (Δ CPM) entre le nombre de coups obtenus en présence d'antigène diminué du nombre de coups en l'absence d'antigène. Les chiffres entre parenthèses indiquent les indices de stimulation, c'est-à-dire le rapport du nombre de coups obtenus en présence d'antigènes sur le nombre de coups obtenus en l'absence d'antigènes.

Les résultats sont considérés comme positifs quand le Δ CPM est supérieur à 1000 et quand l'indice de stimulation est supérieur à 3.

### 4.4. Comparaison des réponses en anticorps de chimpanzés Nuria avant et après immunisation par différents peptides.

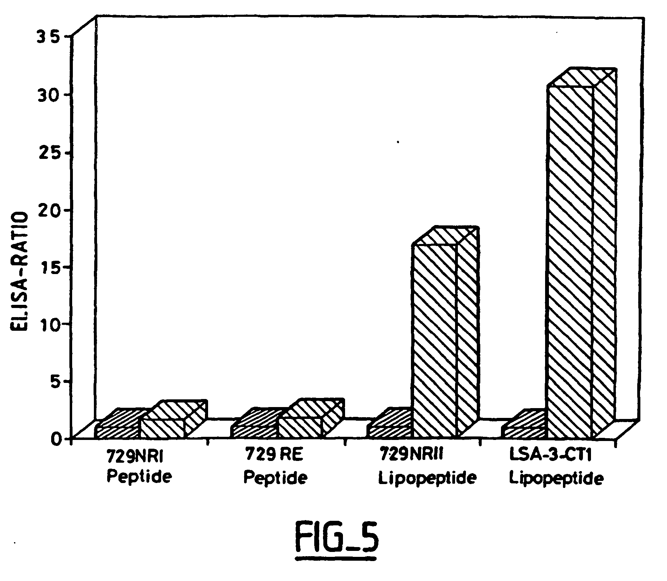

La figure 5 représente les quantités d'immunoglobulines présentes dans le sérum des chimpanzés Nuria avant et après immunisation par les peptides 729NR1 et 729RE, et les lipopeptides 729NR2, et CT1.

Cette expérience montre la supériorité quant à l'immunité B de l'antigène R, surtout quand il est conjugué à un résidu lipidique.

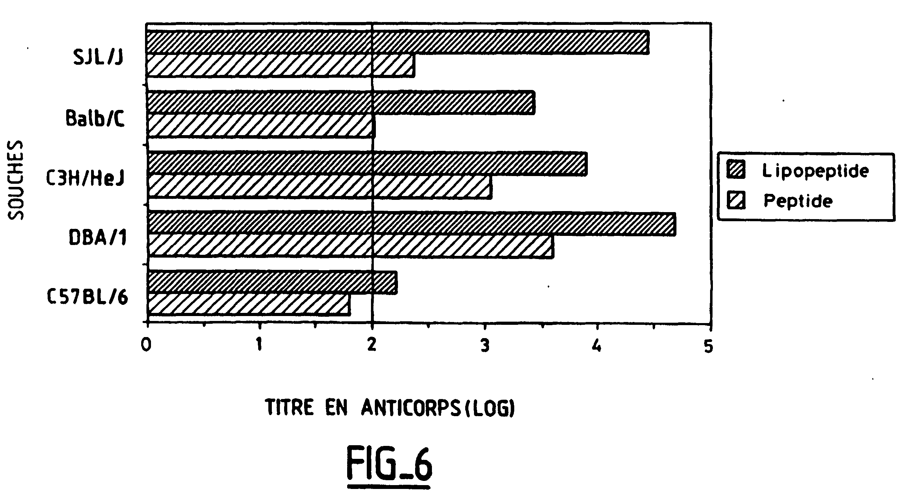

La figure 6 montre que le niveau d'anticorps spécifiques mesuré par Elisa contre le peptide 729NR2 dans des souris immunisées avec soit le peptide 729 NRII soit le lipopeptide 729NRII est nettement supérieur quand le lipopeptide est utilisé quelle que soit l'espèce de souris.

### Exemple 5: Lymphoprolifération des PBMC d'un individu protégé par injection de sporozoïtes irradiés contre des peptides issus des antigènes LSA-1 et LSA-3.

Chez huit volontaires humains immunisés par injection de sporozoïtes irradiés, des anticorps anti-LSA-3 sont trouvés chez chacun des quatre individus résistants à une infection par sporozoïtes ; aucun chez les quatre autres volontaires ayant développé une infection sanguine.

De plus, pour le seul de ces quatre individus protégés dont les cellules étaient accessibles, les PBMC ont été prélevées six mois après l'infection d'épreuve et incubées en présence des peptides issus des antigènes LSA-1 et LSA-3.

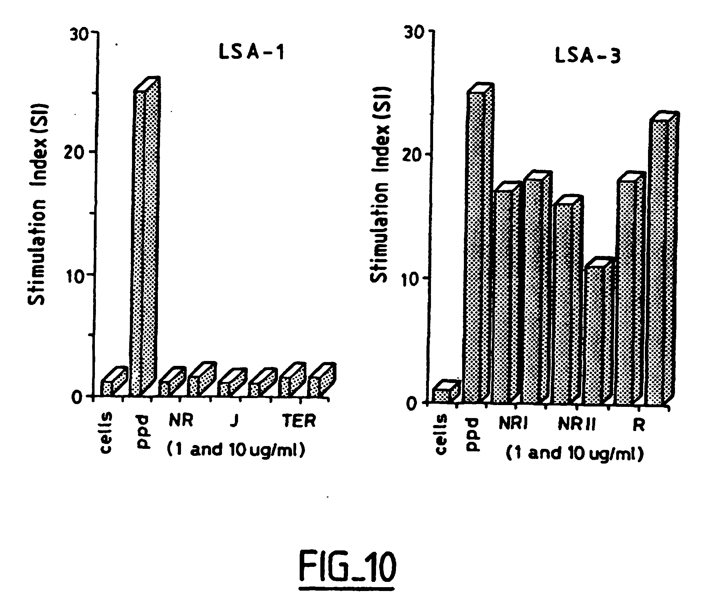

La figure 10 représente les résultats des lymphoproliférations des PBMC d'un individu protégé par injection des sporozoïtes irradiés contre des peptides issus des antigènes LSA-1 et LSA-3.

Des lymphoproliférations importantes ont été observées avec chacun des trois peptides LSA-3 (NR1, NR2 et RE) mais avec aucun des peptides LSA-1. Il existait un niveau particulièrement élevé de sécrétion d'IFN-γ (100 Ul/ml) après stimulation par le peptide NR1 et, à un moindre degré, par le peptide NR2 (IFN-γ : la cytokine ayant le plus fort effet bloquant sur la schizogonie hépatique).

### Exemple 6 : Effets des anticorps contre les peptides de LSA-3 sur l'inhibition de la pénétration des sporozoïtes chez la souris.

Les techniques utilisées pour préparer les cultures primaires d'hépatocytes, les sporozoïtes, les anticorps et le test de fluorescence indirecte sont décrites en détail par S.MELLOUK et al, Bulletin of the World Health Organization, 68 : 52-59, 1990. Le tableau V ci-dessous compare les résultats obtenus en immuno fluorescence soit par des anticorps contre le fragment 679 soit par des anticorps obtenus contre des fragments en provenance d'autres peptides. La colonne de gauche indique le nombre de schizontes détectés après 48h de culture dans des hépatocytes de souris Balb c infectées par P.yoelii et la colonne de droite les mêmes paramètres après infection par P.Berghei.

**Tableau V :**

| Clones d'anticorps | | P.yoelii | | P.berghei | | |
|---|---|---|---|---|---|---|
| | IFA | Nbre de LS à 48 h | | IFA | Nbre de LS à 48 h | |
| | | a) | b) | | | |
| Contrôle | | 88 | 110 | | 119 | 108 |
| 679 | ++ | | 0 | - | | 47 |
| | ++ | | 0 | - | | ND |
| 679 | ++ | 1 | | - | 105 | |
| 679b | ++ | 1 | | - | 133 | |
| 679c | ++ | 1 | | - | 30 | |
| 32 | ++ | 8 | | ± | 103 | |
| 222 | + | | 5 | ± | | 26 |
| 667 | ++ | 276 | 143 | ND | 502 | |
| 362 | + | 3 | | | | |
| 493 α | ++ | 55 | | ND | 508 | |
| P.b. CSP Mab | | | 82 | +++ | | 30 |
| α P.y. CSP Mab | +++ | | 171 | | 138 | |

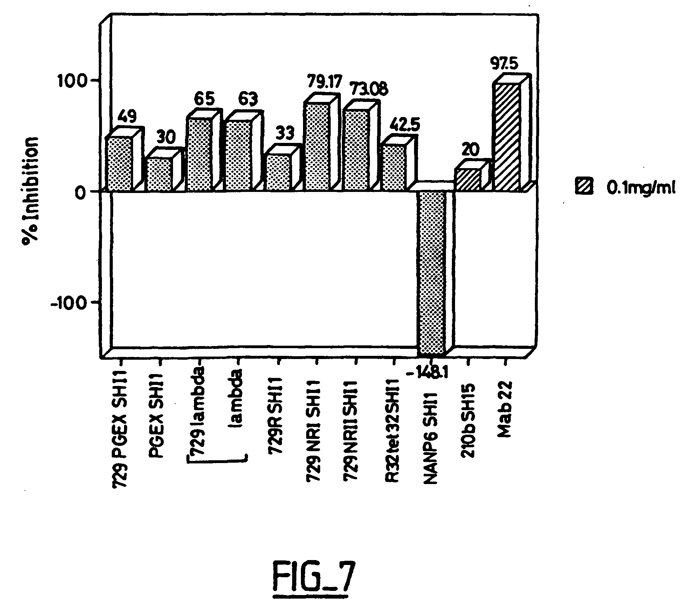

il apparaît clairement que l'anticorps contre le peptide 679 a un effet d'inhibition quasi total du nombre de ce qu'ils ont observés à 48h dans les cellules hépatiques. De la même façon La figure 7 montre l'inhibition de l'invasion des cellules hépatiques par les sporozoïtes par des sérums hyper-immuns obtenus après immunisation par différents peptides et immuno purifiés contre du LSA-3 entier.

En ce qui concerne les protections de souris, les meilleurs résultats ont été obtenus par immunisation avec les recombinants, ou antigènes préparés selon l'invention, adsorbés sur des microsphères de latex ou de polystyrène de 0,5µm de diamètre :
- 3/3 souris sont protégées contre une administration par 10 fois la dose minimale infectieuse
- 3/3 souris sont protégées contre le second challenge
- 2/3 souris sont protégées contre le troisième challenge.

Les microsphères utilisées sont les Polybead ® Polystyrène Microsphères (Polysciences, Inc.) de 0,50 µm de diamètre (réf.07307) sur lesquelles les recombinants ou les peptides sont adsorbés passivement. En pratique, chez la souris, pour 1 injection, 50µg d'antigènes sont mis en contact avec 50µl de microbilles ; la quantité exacte d'antigènes adsorbée n'est pas déterminée. Chez le chimpanzé, la même procédure est effectuée avec 200 µg d'antigènes et 200 µl de billes.
De plus, récemment, l'immunisation de souris par le recombinant GST-3PC (correspondant à la région 3' non-répétée de l'acide aminé n° 869 au codon stop en 3') a permis d'obtenir des sérums réagissant très fortement en immunofluorescence sur les sporozoites de Plasmodium falciparum. Ce résultat est la première démonstration de la présence d'un ou de plusieurs épitopes B dans cette région de la molécule.

### Exemple 7 : test de cytotoxicité contre le peptide 729 NRII chez le chimpanzé Gerda.

Le chimpanzé Gerda a été immunisé par voie i.v. avec le lipopeptide 729NRII issu de l'antigène LSA-3. Le sang est prélevé 9 jours après la 4ème injection. Les PBMC ont été incubés in vitro avec 5µg/ml du peptide 729NRII (ajout de l'IL2 recombinante, 10 U/ml, au jour 3). Au jour 15, l'activité cytotoxique a été étudiée contre les blastes autologues générés par la PHA à 0,5µg/ml. Les blastes ont été préincubés la nuit avec 5 µg/ml du peptide 729NRII, et avec un peptide contrôle : le RESA, ou sans peptide. Les peptides ne sont pas ajoutés pendant le test (8 heures). Le nombre de cibles par puits est de 5000.
Les PBMC de Gerda incubées pendant la même période avec 5µg/ml d'un peptide contrôle ou le peptide 729NRI (issu du même antigène), n'engendrent pas la lyse des blastes autologues préincubés ou non avec les peptides ci-dessus.

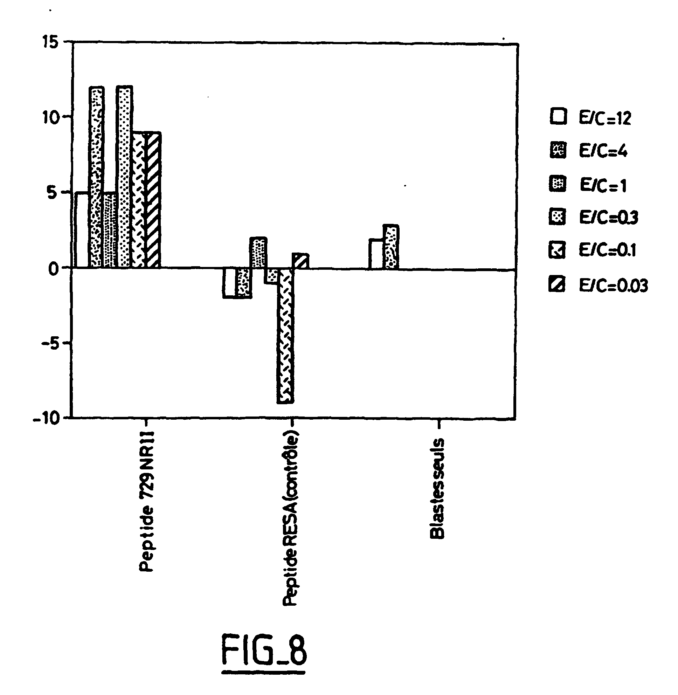

La figure 8 indique les résultats obtenus pour un rapport E/C (effecteur sur cible) variant de 12 à 0,03. On voit que les cellules cibles pré-sensibilisées par le peptide 729NRII sont lysés en présence de cellules effectrices ce qui indique une réponse immunitaire de type T cytotoxiqué spécifique de cet antigène.
Le lipopeptide NRII, injecté par voie IV, est capable, sans adjuvant, d'induire une réponse cytotoxique spécifique.

### Exemple 8 : effet du peptide NRI sur la production d'interféron γ.

Il a été montré que les interférons avaient une activité inhibitrice dans le développement du P.falciparum dans les hépatocytes humains en culture (Sylvie Mellouk et al, The Journal of Immunology, vol 139 n° 12 : 41-92, 41-95, 1987). Les résultats obtenus avec les peptides de l'invention sont les suivants :
Le chimpanzé Gerda, immunisé par la polypeptide NR 2 et boosté par le recombinant DG729, porte des PBMC capables de sécreter des taux élevés d'IFN-γ en présence des peptides LSA-3, en particulier le peptide 729 NR1. Le résultat a été confirmé chez le chimpanzé Dirk, immunisé par la même protéine. Le chimpanzé BRAM, contrôle non immunisé, ne montre aucune interféronémie contre les peptides LSA-3.
## Revendications

1. Molécules polypeptidiques contenant au moins 10 amino acides consécutifs de la séquence d'acides aminés montrée dans la figure 2, les polypeptides suivants étant exclus :
- RDELFNELLNSVDVNGEVKENILEESQVNDDIFNSLVKSVQQEQQHNVEEK VEESVEENDEESVEENVEENVENNDDGSVASSVEESIASSVDESIDSSIE ENVAPTVEEIVAPTVEEIVAPSWEKCAPSVEESVAPSVEESVAEMLKER (729S)
- RDELFNELLNSVDVNGEVKENILEESQVNDDIFNSLVKSVQQEQQHN
- DELFNELLNSVDVNGEVKENILEESQ (NR I),
- LEESQVNDDIFSNSLVKSVQQEQQHNV (NR II),
- VESVAPSVEESVAPSVEESVAENVEESV. (729 RE)

2. Molécules selon la revendication 1, **caractérisées en ce qu'**elles contiennent au moins 20 amino acides consécutifs de ladite séquence.

3. Molécules selon la revendication 2 **caractérisées en ce qu'**elles contiennent au moins 50 amino acides consécutifs de la ladite séquence.

4. Molécule polypeptide présentant au moins 70% d'homologie avec l'une des molécules de l'une quelconque des revendications 1 à 3.

5. Molécule polypeptidique **caractérisée en ce qu'**elle présente au moins 70% d'homologie avec la séquence suivante :

6. Molécule polypeptidique selon l'une des revendications 1 à 4 **caractérisée en ce qu'**elle présente au moins 70% d'homologie avec la séquence représentée dans la figure 3.

7. Composition immunogène **caractérisée en ce qu'**elle contient au moins une molécule polypeptidique selon l'une quelconque des revendications 1 à 6 et au moins un véhicule pharmaceutique.

8. Composition de vaccin anti-palustre, contenant entre autres principes immunogènes, une molécule polypeptidique conforme à l'une des revendications 1 à 6.

9. Composition de vaccin selon la revendication 8, **caractérisée en ce qu'**elle contient en outre , une molécule contenant au moins un épitope et issue du groupe constitué des molécules LSA-1, SALSA ou STARP.

10. Composition selon la revendication 9 **caractérisée en ce qu'**elle contient au moins deux immunogènes, le premier étant choisi parmi les polypeptides suivants :
- celui de la figure 2,
- NRI,
- NRII,
et le deuxième étant choisi dans le groupe constitué de SALSA, SALSA 1 et SALSA 2.

11. Composition de vaccin anti-palustre, **caractérisée en ce qu'**elle contient au moins deux immunogènes, le premier étant choisi parmi les polypeptides suivants :
- celui de la figure 2,
- NRI,
- NRII,
et le deuxième étant choisi dans le groupe constitué de SALSA, SALSA 1 et SALSA 2.

12. Composition selon la revendication 11, **caractérisée en ce qu'**elle contient le polypeptide de la figure 2.

13. Anticorps polyclonaux ou monoclonaux, reconnaissant spécifiquement les molécules polypeptidiques selon l'une quelconque des revendications 1 à 6.

14. Méthode de diagnostic in vitro du paludisme chez un individu susceptible d'être infecté par P.falciparum qui comprend la mise en contact d'un tissu ou d'un fluide biologique prélevé chez un individu, avec une molécule selon l'une des revendications 1 à 8 dans des conditions permettant une réaction immunologique entre ladite molécule polypeptidique et les anticorps éventuellement présents dans le tissu ou le fluide biologique, et la détection in vitro des complexes antigènes / anticorps éventuellement formés.

15. Méthode selon la revendication 14 **caractérisée en ce que** le tissu ou le fluide biologique sont mis en contact avec un mélange de molécules polypeptidiques répondant à l'une des revendications 1 à 6 et d'autres molécules issues d'antigènes du stade sporozoïte que sont LSA-1, SALSA ou STARP.

16. Méthode de diagnostic in vitro du paludisme chez un individu susceptible d'être infecté par P.falciparum, **caractérisée en ce qu'**elle comprend la mise en contact d'un tissu ou d'un fluide biologique prélevé chez un individu, avec des anticorps selon la revendication 13, dans des conditions permettant une réaction immunologique in vitro entre lesdits anticorps et les protéines spécifiques de P.falciparum éventuellement présentes dans le tissu biologique, et la détection in vitro des complexes antigènes anticorps éventuellement formés.

17. Kit pour le diagnostic in vitro du paludisme selon la revendication 14 ou 15 **caractérisé en ce qu'**il comprend au moins une ou plusieurs molécules selon l'une des revendications 1 à 6,
les réactifs pour la constitution du milieu propice à la réaction,
les réactifs permettant la détection des complexes antigènes/anticorps produits par la réaction immunologique, ces réactifs pouvant également porter un marqueur ou être susceptibles d'être reconnus à leur tour par un réactif marqué, plus particulièrement dans le cas où la molécule polypeptidique susmentionnée n'est pas marquée.

18. Kit pour le diagnostic in vitro du paludisme **caractérisé en ce qu'**il comprend :
- des anticorps selon la revendication 13,
- les réactifs pour la constitution du milieu propice à la réalisation de la réaction immunologique,
- les réactifs permettant la détection des complexes antigènes/anticorps produits par la réaction immunologique, ces réactifs pouvant également porter un marqueur ou être susceptibles d'être reconnus à leur tour par un réactif marqué, plus particulièrement dans le cas où les anticorps susmentionnés ne sont pas marqués.

19. Utilisation d'une molécule polypeptidique selon l'une des revendications 1 à 6 dans la préparation d'un vaccin anti-palustre.

20. Utilisation d'un ou plusieurs anticorps polyclonaux ou monoclonaux selon la revendication 13 pour la préparation d'un médicament destiné au traitement du paludisme.

21. Composition pharmaceutique comportant à titre de substance active un ou plusieurs anticorps polyclonaux ou monoclonaux selon la revendication 13 en association avec un véhicule pharmaceutique acceptable.

22. Séquence d'acides nucléiques **caractérisée par** l'une des séquence suivantes :
(a) l'enchaînement de nucléotides tel que représenté dans SEQ ID n° 1 de la figure 1, ou
(b) l'enchaînement de nucléotides représenté dans SEQ ID n° 2 de la figure 2,
(c) un enchaînement présentant au moins 70% d'homologie avec celui de la figure 1, ou de la figure 2 ou,
(d) un enchaînement de nucléotides complémentaires à ceux présentés en (a), (b) ou (c).

23. Acide nucléique selon la revendication 22 contenant une séquence codant pour une molécule polypeptidique selon l'une des revendications 1 à 6.

24. Vecteur recombinant pour le clonage d'une séquence nucléotidique selon la revendication 22 ou 23 et/ou l'expression d'un polypeptide codé par la susdite séquence contenant ladite séquence dans l'un des sites non essentiels pour sa réplication, ledit vecteur étant notamment de type plasmide, cosmide ou phage.

25. Vecteur selon la revendication 24 **caractérisé en ce qu'**il est un plasmide déposé à la CNCM sous le n°I-1573 et référencé pK1.2.

26. Conjugués constitués de molécules polypeptidiques selon l'une quelconque des revendications 1 à 6 et d'un support sur lequel lesdites molécules sont adsorbées.

27. Conjugués selon la revendication 26 **caractérisés en ce que** le support est constitué de microsphères ou billes de latex ou de polystyrène.

28. Utilisation d'un conjugué selon l'une des revendications 26 ou 27, pour la préparation d'une composition destinée à l'immunisation d'individus infectés ou susceptibles d'être infectés par le paludisme.

## Patentansprüche

1. Polpeptidmoleküle, enthaltend wenigstens 10 aufeinander folgende Aminosäuren der in der Figur 2 gezeigten Aminosäuresequenz, wobei die folgenden Polypeptide ausgenommen sind:
- RDELFNELLNSVDVNGEVKENILEESQVNDDIFNSLVKSVQQEQQH NVEEKVEESVEENDEESVEENVEENVENNDOGSVASSVEESIASS VDESIDSSIEENVAPTVEEIVAPTVEEIVAPSVVEKCAPSVEESVAPS VEESVAEMLKER (729S)
- RDELFNELLNSVDVNGEVKENILEESQVNDDIFNSLVKSVQQEQQHN
- DELFNELLNSVDVNGEVKENILEESQ (NR I),
- LEESQVNDDIFSNSLVKSVQQEQQHNV (NR II),
- VESVAPSVEESVAPSVEESVAENVEESV. (729 RE)

2. Moleküle nach Anspruch 1, **dadurch gekennzeichnet, dass** sie wenigstens 20 aufeinander folgende Aminosäuren der besagten Sequenz enthalten.

3. Moleküle nach Anspruch 2, **dadurch gekennzeichnet, dass** sie wenigstens 50 aufeinander folgende Aminosäuren der besagten Sequenz enthalten.

4. Polypeptidmolekül, das wenigstens 70 % Homologie mit einem der Moleküle eines beliebigen der Ansprüche 1 bis 3 aufweist.

5. Polypeptidmolekül, **dadurch gekennzeichnet, dass** es wenigstens 70 % Homologie mit der folgenden Sequenz aufweist:

6. Polypeptidmolekül nach einem der Ansprüche 1 bis 4, **dadurch gekennzeichnet, dass** es wenigstens 70 % Homologie mit der in der Figur 3 dargestellten Sequenz aufweist.

7. Immunogene Zusammensetzung, **dadurch gekennzeichnet, dass** sie wenigstens ein Polypeptidmolekül nach einem beliebigen der Ansprüche 1 bis 6 und wenigstens einen pharmazeutischen Träger enthält.

8. Anti-Malaria-Impfstoffzusammensetzung, enthaltend unter anderen immunogenen Wirkstoffen ein Polypeptidmolekül gemäß einem der Ansprüche 1 bis 6.

9. Impfstoffzusammensetzung nach Anspruch 8, **dadurch gekennzeichnet, dass** sie unter anderem ein Molekül enthält, das wenigstens ein Epitop enthält und von der aus den Molekülen LSA-1, SALSA oder STARP bestehenden Gruppe abstammt.

10. Zusammensetzung nach Anspruch 9, **dadurch gekennzeichnet, dass** sie wenigstens zwei Immunogene enthält, das erste, das unter den folgenden Polypeptiden ausgewählt ist:
- dem der Figur 2,
- NRI,
- NRII,
und das zweite, das aus der Gruppe, bestehend aus SALSA, SALSA 1 und SALSA 2, ausgewählt ist.

11. Anti-Malaria-Impfstoffzusammensetzung, **dadurch gekennzeichnet, dass** sie wenigstens zwei Immunogene enthält, das erste, das unter den folgenden Polypeptiden ausgewählt ist:
- dem der Figur 2,
- NRI,
- NRII,
und das zweite, das aus der Gruppe, bestehend aus SALSA, SALSA 1 und SALSA 2, ausgewählt ist.

12. Zusammensetzung nach Anspruch 11, **dadurch gekennzeichnet, dass** sie das Polypeptid der Figur 2 enthält.

13. Polyklonale oder monoklonale Antikörper, die spezifisch die Polypeptidmoleküle nach einem beliebigen der Ansprüche 1 bis 6 erkennen.

14. Verfahren zur in-vitro-Diagnose von Malaria bei einem Individuum, das empfänglich dafür ist, von P. falciparum infiziert zu werden, umfassend das In-Kontakt-Bringen eines Gewebes oder einer biologischen Flüssigkeit, die aus einem Individuum entnommen wurden, mit einem Molekül nach einem der Ansprüche 1 bis 8 unter Bedingungen, die eine Immunreaktion zwischen dem besagten Polypeptidmolekül und den möglicherweise in dem Gewebe oder der biologischen Flüssigkeit vorhandenen Antikörpern erlauben, und den in-vitro-Nachweis von möglicherweise gebildeten Antigen/Antikörper-Komplexen.

15. Verfahren nach Anspruch 14, **dadurch gekennzeichnet, dass** das Gewebe oder die biologische Flüssigkeit mit einem Gemisch von Polypeptidmolekülen gemäß einem der Ansprüche 1 bis 6 und anderen Molekülen, die von Antigenen des Sporozoitenstadium, die LSA-1, SALSA oder STARP sind, abstammen, in Kontakt gebracht wird.

16. Verfahren zur in-vitro-Diagnose von Malaria bei einem Individuum, das empfänglich dafür ist, von P. falciparum infiziert zu werden, **dadurch gekennzeichnet, dass** es das In-Kontakt-Bringen eines Gewebes oder einer biologischen Flüssigkeit, die aus einem Individuum entnommen wurden, mit Antikörpern gemäß Anspruch 13 unter Bedingungen, die eine Immunreaktion in-vitro zwischen den besagten Antikörpern und den möglicherweise in dem biologischen Gewebe vorhandenen, für P. falciparum spezifischen Proteinen erlauben, und den in-vitro-Nachweis von möglicherweise gebildeten Antigen/Antikörper-Komplexen umfasst.

17. Kit für die in-vitro-Diagnose von Malaria nach Anspruch 14 oder 15, **dadurch gekennzeichnet, dass** es wenigstens eines oder mehrere Moleküle nach einem der Ansprüche 1 bis 6,
die Reagenzien für die Bildung des günstigen Milieus für die Reaktion,
die Reagenzien, die den Nachweis von durch die Immunreaktion gebildeten Antigen/Antikörper-Komplexen erlauben, umfasst, wobei diese Reagenzien in gleicher Weise eine Markierung tragen oder geeignet sein können, ihrerseits von einem markierten Reagens erkannt zu werden, insbesondere falls das oben genannte Polypeptidmolekül nicht markiert ist.

18. Kit für die in-vitro-Diagnose von Malaria, **dadurch gekennzeichnet, dass** es umfasst:
- Antikörper nach Anspruch 13,
- die Reagenzien für die Bildung des günstigen Milieus für die Durchführung der Immunreaktion,
- die Reagenzien, die den Nachweis von durch die Immunreaktion gebildeten Antigen/Antikörper-Komplexen erlauben, wobei diese Reagenzien in gleicher Weise eine Markierung tragen oder geeignet sein können, ihrerseits von einem markierten Reagens erkannt zu werden, insbesondere falls die oben genannten Antikörper nicht markiert sind.

19. Verwendung eines Polypeptidmoleküls nach einem der Ansprüche 1 bis 6 bei der Herstellung eines Anti-Malaria-Impfstoffs.

20. Verwendung eines oder mehrerer polyklonaler oder monoklonaler Antikörper nach Anspruch 13 für die Herstellung eines zur Behandlung der Malaria bestimmten Medikaments.

21. Pharmazeutische Zusammensetzung, enthaltend als aktive Substanz einen oder mehrere polyklonale oder monoklonale Antikörper nach Anspruch 13 gemeinsam mit einem pharmazeutisch annehmbaren Trägermedium.

22. Nukleinsäuresequenz, **gekennzeichnet durch** eine der folgenden Sequenzen:
(a) die wie in SEQ ID NR. 1 der Figur 1 gezeigte Nukleotidkette, oder
(b) die in SEQ ID NR. 2 der Figur 2 gezeigte Nukleotidkette,
(c) eine Kette, die wenigstens 70 % Homologie mit derjenigen der Figur 1 oder der Figur 2 zeigt, oder
(d) eine Kette komplementärer Nukleotide zu den in (a), (b) oder (c) dargestellten.

23. Nukleinsäure nach Anspruch 22, enthaltend eine für ein Polypeptidmolekül nach einem der Ansprüche 1 bis 6 kodierende Sequenz.

24. Rekombinanter Vektor für das Klonieren einer Nukleotidsequenz nach Anspruch 22 oder 23 und/oder die Expression eines von der oben genannten Sequenz kodierten Polypeptids, der die besagte Sequenz in einer der für seine Replikation nicht essentiellen Stellen enthält, wobei der besagte Vektor insbesondere vom Typ Plasmid, Cosmid oder Phage ist.

25. Vektor nach Anspruch 24, **dadurch gekennzeichnet, dass** er ein bei der CNCM unter der Nr. I-1573 hinterlegtes und mit pK1.2 bezeichnetes Plasmid ist.

26. Konjugate, bestehend aus Polypeptidmolekülen nach einem beliebigen der Ansprüche 1 bis 6 und einem Träger, auf dem die besagten Moleküle adsorbiert sind.

27. Konjugate nach Anspruch 26, **dadurch gekennzeichnet, dass** der Träger aus Mikrosphären oder Kügelchen aus Latex oder aus Polystyrol besteht.

28. Verwendung eines Konjugats nach einem der Ansprüche 26 oder 27 für die Herstellung einer für die Immunisierung von Individuen, die mit Malaria infiziert sind oder empfänglich dafür sind, mit Malaria infiziert zu werden, bestimmten Zusammensetzung.

## Claims

1. Polypeptide molecules containing at least 10 consecutive amino acids of the amino acid sequence shown in Figure 2, the following polypeptides being excluded:
- RDELFNELLNSVDVNGEVKENILEESQVNDDIFNSLVKSVQQEQQHNVEE KVEESVEENDEESVEENVEENVENNDDGSVASSVEESIASSVDESIDSSIEE NVAPTVEEIVAPTVEEIVAPSWEKCAPSVEESVAPSVEESVAEMLKER (729S),
- RDELFNELLNSVDVNGEVKENILEESQVNDDIFNSLVKSVQQEQQHN,
- DELFNELLNSVDVNGEVKENILEESQ, (NRI)
- LEESQVNDDIFSNSLVKSVQQEQQHNV, (NRII)
- VESVAPSVEESVAPSVEESVAENVESSV. (729RE)

2. Molecules according to Claim 1, **characterized in that** they contain at least 20 consecutive amino acids of said sequence.

3. Molecules according to Claim 2, **characterized in that** they contain at least 50 consecutive amino acids of said sequence.

4. Polypeptide molecule displaying at least 70% homology with one of the molecules of anyone of Claims 1 to 3.

5. Polypeptide molecule, **characterized in that** it displays at least 70% homology with the following sequence:

6. Polypeptide molecule according to one of Claims 1 to 4, **characterized in that** it displays at least 70% homology with the sequence depicted in Figure 3.

7. Immunogenic composition, **characterized in that** it contains at least one polypeptide molecule according to any one of Claims 1 to 6 and at least one pharmaceutical vehicle.

8. Antimalarial vaccine composition containing, among other immunogenic principles, a polypeptide molecule according to one of Claims 1 to 6.

9. Vaccine composition according to Claim 8, **characterized in that** it contains, in addition, a molecule containing at least one epitope and which originates from the group consisting of the LSA-1, SALSA or STARP molecules.

10. Composition according to Claim 9, **characterized in that** it contains at least two immunogens, the first being chosen from the following polypeptides:
- that of Figure 2,
- NRI,
- NRII,
and the second being chosen from the group consisting of SALSA, SALSA I and SALSA II.

11. Antimalarial vaccine composition **characterized in that** it contains at least two immunogens, the first one being chosen from the following polypeptides:
- that of Figure 2,
- NRI,
- NRII
and the second being chosen from the group consisting of SALSA, SALSA I and SALSA II.

12. Composition according to claim 11, **characterized in that** it contains the polypeptide of Figure 2.

13. Polyclonal or monoclonal antibodies which specifically recognize the polypeptide molecules according to any one of Claims 1 to 6.

14. Method of in vitro diagnosis of malaria in an individual likely to be infected by P. falciparum, which comprises the bringing of a tissue or biological fluid taken from an individual, into contact with a molecule according to one of Claims 1 to 8, under conditions enabling an immunological reaction, between said polypeptide molecule and the antibodies possibly present in the tissue or the biological fluid, and the in vitro detection of the antigen/antibody complexes possibly formed.

15. Method according to Claim 14, **characterized in that** the tissue or biological fluid is brought into contact with a mixture of polypeptide molecules corresponding to one of Claims 1 to 6 and other molecules originating from antigens of the sporozoite stage, namely LSA-1, SALSA or STARP.

16. Method of in vitro diagnosis of malaria in an individual likely to be infected by P. falciparum, **characterized in that** it comprises the bringing of a tissue or biological fluid taken from an individual into contact with antibodies according to Claim 13, under conditions enabling an in vitro immunological reaction between said antibodies and the P. falciparum specific proteins which are possibly present in the biological tissue, and the in vitro detection of the antigen/antibody complexes possibly formed.

17. Kit for the in vitro diagnosis of malaria according to Claim 14 or 15, **characterized in that** it comprises at least one or several molecules according to one of Claims 1 to 6,
the reagents for making up the appropriate medium for the reaction,
the reagents enabling the detection of antigen/antibody complexes produced by the immunological reaction, these reagents optionally carrying a label or being capable of being recognized in their turn by a labelled reagent, more especially in the case where the abovementioned polypeptide molecule is not labelled.

18. Kit for the in vitro diagnosis of malaria, **characterized in that** it comprises:
- antibodies according to Claim 13,
- the reagents for making up the appropriate medium for carrying out the immunological reaction,
- the reagents enabling the detection of antigen/antibody complexes produced by the immunological reaction, these reagents optionally carrying a label or being capable of being recognized in their turn by a labelled reagent, more especially in the case where the abovementioned antibodies are not labelled.

19. Use of a polypeptide molecule according to one of Claims 1 to 6 in the preparation of an antimalarial vaccine.

20. Use of one or more polyclonal or monoclonal antibodies according to Claim 13 for the preparation of a medicament for the treatment of malaria.

21. Pharmaceutical composition containing as active substance one or more polyclonal or monoclonal antibodies according to Claim 13, in combination with an acceptable pharmaceutical vehicle.

22. Nucleic acid sequence, **characterized by** one of the following sequences:
(a) the linked succession of nucleotides as depicted in SEQ ID No. 1 of Figure 1, or
(b) the linked succession of nucleotides depicted in SEQ ID No. 2 of Figure 2,
(c) a linked succession displaying at least 70% homology with that of Figure 1 or of Figure 2, or
(d) a linked succession of nucleotides which are complementary to those presented in (a), (b) or (c).

23. Nucleic acid according to Claim 22, containing a sequence encoding a polypeptide molecule according to one of Claims 1 to 6.

24. Recombinant vector, for the cloning of a nucleotide sequence according to Claim 22 or Claim 23 and/or the expression of a polypeptide encoded by the abovementioned sequence, containing said sequence in one of the non-essential sites for its replication, said vector being, in particular, of the plasmid, cosmid or phage type.

25. Vector according to Claim 24, **Characterized in that** it is a plasmid deposited at the CNCM under the No. I-1573 and referenced pK1.2.

26. Conjugates consisting of polypeptide molecules according to any one of Claims 1 to 6 and a support on which said molecules are adsorbed.

27. Conjugates according to Claim 26, **characterized in that** the support consists of latex or polystyrene microspheres or beads.

28. Use of a conjugate according to one of Claims 26 and 27 in the preparation of a composition for the immunization of individuals who are infected or likely to be infected with malaria.


(2) INFORMATIONS POUR LA SEQ ID NO: 4:
   (i) CARACTERISTIQUES DE LA SEQUENCE:
      (A) LONGUEUR: 28 paires de bases
      (B) TYPE: nucléotide
      (C) NOMBRE DE BRINS: simple
      (D) CONFIGURATION: linéaire
   (ii) TYPE DE MOLECULE: ADN (génomique)
   (xi) DESCRIPTION DE LA SEQUENCE: SEQ ID NO: 4:
   GTGATGAACT TTTTAATGAA TTATTAAA   28
(2) INFORMATIONS POUR LA SEQ ID NO: 5:
   (i) CARACTERISTIQUES DE LA SEQUENCE:
      (A) LONGUEUR: 29 paires de bases
      (B) TYPE: nucléotide
      (C) NOMBRE DE BRINS: simple
      (D) CONFIGURATION: linéaire
   (ii) TYPE DE MOLECULE: ADN (génomique)
   (xi) DESCRIPTION DE LA SEQUENCE: SEQ ID NO: 5:
   TGTTGTTCTT GTTGAACACT TTTTACTAA 29
(2) INFORMATIONS POUR LA SEQ ID NO: 6:
   (i) CARACTERISTIQUES DE LA SEQUENCE:
      (A) LONGUEUR: 25 paires de bases
      (B) TYPE: nucléotide
      (C) NOMBRE DE BRINS: simple
      (D) CONFIGURATION: linéaire
   (ii) TYPE DE MOLECULE: ADN (génomique)
   (xi) DESCRIPTION DE LA SEQUENCE: SEQ ID NO: 6:
   GGTATCGAAA CTGAGGAAAT AAAGG 25
(2) INFORMATIONS POUR LA SEQ ID NO: 7:
   (i) CARACTERISTIQUES DE LA SEQUENCE:
      (A) LONGUEUR: 24 paires de bases
      (B) TYPE: nucléotide
      (C) NOMBRE DE BRINS: simple
      (D) CONFIGURATION: linéaire
   (ii) TYPE DE MOLECULE: ADN (génomique)
   (xi) DESCRIPTION DE LA SEQUENCE: SEQ ID NO: 7:
   CATAGCAGGA ACATCAACAT CCAC 24